# EUROPEAN PATENT APPLICATION

(11) **EP 3 878 858 A1**
(43) Date of publication of application: **15.09.2021**
(21) Application number: 20305255.0
(22) Date of filing: 11.03.2020
(51) Int. Cl.: C07K 14/195, C12N 15/10

(54) **VARIANTS OF SAC7D AND THEIR USE IN CANCER THERAPY**

(71) Applicant: AFFILOGIC, 44200 Nantes (FR)
(72) Inventor: The designation of the inventor has not yet been filed
(74) Representative: Flesselles, Bruno F.G.

(57) **Abstract**

The invention relates to variants of OB-fold proteins, in particular of the Sac7d family that bind PD-L1, HSP110 or EGFR and are able to be used alone or in combination for cancer treatment.

SEQUENCE LISTING

<110>  AFFILOGIC

<120>  METHODS OF TREATING CANCER

<130>  BRV 173-EP PRIO

<160>  29

<170>  PatentIn version 3.5

<210>  1
<211>  66
<212>  PRT
<213>  Sulfolobus acidocaldarius

<400>  1

Met Val Lys Val Lys Phe Lys Tyr Lys Gly Glu Glu Lys Glu Val Asp
1               5                   10                  15

Thr Ser Lys Ile Lys Lys Val Trp Arg Val Gly Lys Met Val Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Leu Asp Met Leu Ala Arg Ala Glu Arg Glu
    50                  55                  60

Lys Lys
65

<210>  2
<211>  64
<212>  PRT
<213>  Sulfolobus solfataricus

<400>  2

Met Ala Thr Val Lys Phe Lys Tyr Lys Gly Glu Glu Lys Glu Val Asp
1               5                   10                  15

Ile Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  3

<211>  65
<212>  PRT
<213>  Sulfolobus acidocaldarius

<400>  3

Met Ala Lys Val Arg Phe Lys Tyr Lys Gly Glu Glu Lys Glu Val Asp
1               5                   10                  15

Thr Ser Lys Ile Lys Lys Val Trp Arg Val Gly Lys Met Val Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Met Asp Met Leu Ala Arg Ala Glu Lys Lys
    50                  55                  60

Lys
65

<210>  4
<211>  64
<212>  PRT
<213>  Sulfolobus shibatae

<400>  4

Met Val Thr Val Lys Phe Lys Tyr Lys Gly Glu Glu Lys Glu Val Asp
1               5                   10                  15

Thr Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  5
<211>  64
<212>  PRT
<213>  Sulfolobus shibatae

<400>  5

Met Ala Thr Val Lys Phe Lys Tyr Lys Gly Glu Glu Lys Gln Val Asp
1               5                   10                  15

Ile Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  6
<211>  64
<212>  PRT
<213>  Sulfolobus tokodaii

<400>  6

Met Val Thr Val Lys Phe Lys Tyr Lys Gly Glu Glu Lys Glu Val Asp
1               5                   10                  15

Ile Ser Lys Ile Lys Lys Val Trp Arg Val Gly Lys Met Ile Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Leu Gln Met Leu Glu Lys Ser Gly Lys Lys
    50                  55                  60

<210>  7
<211>  64
<212>  PRT
<213>  Sulfolobus islandicus

<400>  7

Met Val Thr Val Lys Phe Lys Tyr Lys Gly Glu Glu Lys Gln Val Asp
1               5                   10                  15

Thr Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  8
<211>  64
<212>  PRT
<213>  Sulfolobus islandicus

<400>  8

Met Thr Thr Val Lys Phe Lys Tyr Lys Gly Glu Glu Lys Gln Val Asp

1               5                   10                  15

Thr Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  9
<211>  64
<212>  PRT
<213>  Sulfolobus islandicus

<400>  9

Met Thr Thr Val Lys Phe Lys Tyr Lys Gly Glu Glu Lys Gln Val Asp
1               5                   10                  15

Ile Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  10
<211>  62
<212>  PRT
<213>  Metallosphaera sedula.

<400>  10

Met Ala Thr Lys Ile Lys Phe Lys Tyr Lys Gly Gln Asp Leu Glu Val
1               5                   10                  15

Asp Ile Ser Lys Val Lys Lys Val Trp Lys Val Gly Lys Met Val Ser
            20                  25                  30

Phe Thr Tyr Asp Asp Asn Gly Lys Thr Gly Arg Gly Ala Val Ser Glu
        35                  40                  45

Lys Asp Ala Pro Lys Glu Leu Leu Asn Met Ile Gly Lys Lys
    50                  55                  60

<210>  11
<211>  62

<212>  PRT
<213>  Metallosphaera cuprina

<400>  11

Met Ala Thr Lys Ile Lys Phe Lys Tyr Lys Gly Gln Asp Leu Glu Val
1               5                   10                  15

Asp Ile Ser Lys Val Lys Lys Val Trp Lys Val Gly Lys Met Val Ser
            20                  25                  30

Phe Thr Tyr Asp Asp Asn Gly Lys Thr Gly Arg Gly Ala Val Ser Glu
        35                  40                  45

Lys Asp Ala Pro Lys Glu Leu Leu Ser Met Ile Gly Lys Lys
    50                  55                  60

<210>  12
<211>  61
<212>  PRT
<213>  Acidianus hospitalis

<400>  12

Met Thr Thr Val Lys Phe Lys Tyr Lys Gly Glu Glu Lys Glu Val Asp
1               5                   10                  15

Ile Ser Lys Ile Lys Lys Val Trp Arg Val Gly Lys Met Ile Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Leu Glu Lys Leu Glu Lys Lys
    50                  55                  60

<210>  13
<211>  62
<212>  PRT
<213>  Acidianus hospitalis

<400>  13

Met Ala Thr Lys Val Lys Phe Lys Tyr Lys Gly Glu Glu Lys Glu Val
1               5                   10                  15

Asp Ile Ser Lys Ile Lys Lys Val Trp Arg Val Gly Lys Met Ile Ser
            20                  25                  30

Phe Thr Tyr Asp Asp Asn Gly Lys Thr Gly Arg Gly Ala Val Ser Glu
        35                  40                  45

Lys Asp Ala Pro Lys Glu Leu Leu Asp Lys Leu Glu Lys Lys
    50                  55                  60

<210>  14
<211>  60
<212>  PRT
<213>  Acidianus hospitalis

<400>  14

Met Ala Thr Lys Val Lys Phe Lys Tyr Lys Gly Glu Glu Lys Glu Val
1               5                   10                  15

Asp Ile Ser Lys Ile Lys Lys Val Trp Arg Val Gly Lys Met Ile Ser
            20                  25                  30

Phe Thr Tyr Asp Asp Asn Gly Lys Thr Gly Arg Gly Ala Val Ser Glu
        35                  40                  45

Lys Asp Ala Pro Lys Glu Leu Leu Glu Lys Leu Lys
    50                  55                  60

<210>  15
<211>  68
<212>  PRT
<213>  Artificial Sequence

<220>
<223>  Consensus sequence

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (15)..(15)
<223>  Xaa is E or Q

<220>
<221>  VARIANT

<222>  (18)..(18)
<223>  Xaa is T or I

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(67)
<223>  Xaa is -------, EKS--GK, EK---QK, ARAEREK, ARA-EKK, E-----K,
       EKS--G, ACAEREK oR ACA-EKK

<400>  15

Met Xaa Xaa Xaa Val Xaa Phe Lys Tyr Lys Gly Glu Glu Lys Xaa Val
1               5                   10                  15

Asp Xaa Ser Lys Ile Lys Lys Val Trp Arg Val Gly Lys Met Xaa Ser
            20                  25                  30

Phe Thr Tyr Asp Xaa Xaa Xaa Gly Lys Thr Gly Arg Gly Ala Val Ser
        35                  40                  45

Glu Lys Asp Ala Pro Lys Glu Leu Xaa Xaa Xaa Leu Xaa Xaa Xaa Xaa
    50                  55                  60

Xaa Xaa Xaa Lys
65

<210>  16
<211>  68
<212>  PRT
<213>  Artificial Sequence

<220>
<223>  CONSENSUS SEQUENCE BINDING TO PD-L1 WITH ESSENTIAL AMINO ACIDS

FOR HUMAN PROTEIN BINDING

<220>
<221> VARIANT
<222> (2)..(2)
<223> Xaa is V, A or T

<220>
<221> VARIANT
<222> (3)..(3)
<223> Xaa is T or K

<220>
<221> VARIANT
<222> (4)..(4)
<223> Xaa is - or K

<220>
<221> VARIANT
<222> (6)..(6)
<223> Xaa is R or K

<220>
<221> VARIANT
<222> (8)..(8)
<223> Xaa is any amino acid

<220>
<221> VARIANT
<222> (10)..(10)
<223> Xaa is any amino acid

<220>
<221> VARIANT
<222> (15)..(15)
<223> Xaa is E or Q

<220>
<221> VARIANT
<222> (18)..(18)
<223> Xaa is T or I

<220>
<221> VARIANT
<222> (22)..(23)
<223> Xaa is any amino acid

<220>
<221> VARIANT
<222> (25)..(25)
<223> Xaa is any amino acid

<220>
<221> VARIANT
<222> (30)..(30)
<223> Xaa is any amino acid

<220>
<221> VARIANT
<222> (31)..(31)
<223> Xaa is V or I

<220>
<221>  VARIANT
<222>  (34)..(34)
<223>  Xaa is any amino acid

<220>
<221>  VARIANT
<222>  (37)..(39)
<223>  Xaa Xaa Xaa is EGG or DN-

<220>
<221>  VARIANT
<222>  (42)..(42)
<223>  Xaa is any amino acid

<220>
<221>  VARIANT
<222>  (48)..(48)
<223>  Xaa is any amino acid

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(67)
<223>  Xaa is -------, EKS--GK, EK---QK, ARAEREK, ARA-EKK, E-----K,
       EKS--G, ACAEREK or ACA-EKK

<400>  16

Met Xaa Xaa Xaa Val Xaa Phe Xaa Ile Xaa Gly Glu Glu Lys Xaa Val
1               5                   10                  15

Asp Xaa Ser Lys Ile Xaa Xaa Val Xaa Arg Ile Gly Lys Xaa Xaa Leu
            20                  25                  30

Phe Xaa Tyr Asp Xaa Xaa Xaa Gly Lys Xaa Gly Met Gly Leu Val Xaa
        35                  40                  45

Glu Lys Asp Ala Pro Lys Glu Leu Xaa Xaa Xaa Leu Xaa Xaa Xaa Xaa
    50                  55                  60

Xaa Xaa Xaa Lys
65

<210>  17
<211>  68
<212>  PRT
<213>  Artificial Sequence

<220>
<223>  CONSENSUS SEQUENCE BINDING TO PD-L1 WITH ESSENTIAL AMINO ACIDS
       FOR MOUSE PROTEIN BINDING


<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (8)..(8)
<223>  Xaa is any amino acid

<220>
<221>  VARIANT
<222>  (10)..(10)
<223>  Xaa is any amino acid

<220>
<221>  VARIANT
<222>  (15)..(15)
<223>  Xaa is E or Q

<220>
<221>  VARIANT
<222>  (18)..(18)
<223>  Xaa is T or I

<220>
<221>  VARIANT
<222>  (22)..(23)
<223>  Xaa is any amino acid

<220>
<221>  VARIANT
<222>  (31)..(31)
<223>  Xaa is V or I

<220>
<221>  VARIANT
<222>  (37)..(39)

<223>  Xaa Xaa Xaa is EGG or DN-

<220>
<221>  VARIANT
<222>  (42)..(42)
<223>  Xaa is any amino acid

<220>
<221>  VARIANT
<222>  (48)..(48)
<223>  Xaa is any amino acid

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(67)
<223>  Xaa is -------, EKS--GK, EK---QK, ARAEREK, ARA-EKK, E-----K,
      EKS--G, ACAEREK or ACA-EKK

<400>  17

Met Xaa Xaa Xaa Val Xaa Phe Xaa Ile Xaa Gly Glu Glu Lys Xaa Val
1               5                   10                  15

Asp Xaa Ser Lys Ile Xaa Xaa Val Arg Arg Ile Gly Lys Tyr Xaa Leu
            20                  25                  30

Phe Lys Tyr Asp Xaa Xaa Xaa Gly Lys Xaa Gly Met Gly Leu Val Xaa
        35                  40                  45

Glu Lys Asp Ala Pro Lys Glu Leu Xaa Xaa Xaa Leu Xaa Xaa Xaa Xaa
    50                  55                  60

Xaa Xaa Xaa Lys
65

<210>  18
<211>  68
<212>  PRT
<213>  Artificial Sequence

<220>
<223>  CONSENSUS SEQUENCE BINDING TO PD-L1

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (15)..(15)
<223>  Xaa is E or Q

<220>
<221>  VARIANT
<222>  (18)..(18)
<223>  Xaa is T or I

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(67)
<223>  Xaa is -------, EKS--GK, EK---QK, ARAEREK, ARA-EKK, E-----K,
      EKS--G, ACAEREK or ACA-EKK

<400>  18

Met Xaa Xaa Xaa Val Xaa Phe Val Ile Gly Gly Glu Glu Lys Xaa Val
1               5                   10                  15

Asp Xaa Ser Lys Ile Lys Thr Val Arg Arg Ile Gly Lys Tyr Xaa Leu
            20                  25                  30

Phe Lys Tyr Asp Xaa Xaa Xaa Gly Lys Thr Gly Met Gly Leu Val Ser
        35                  40                  45

Glu Lys Asp Ala Pro Lys Glu Leu Xaa Xaa Xaa Leu Xaa Xaa Xaa Xaa
    50                  55                  60

Xaa Xaa Xaa Lys
65

<210>  19
<211>  66
<212>  PRT
<213>  Artificial Sequence

<220>
<223>  SAC7D VARIANT BINDING TO PD-L1 WITH AMINO ACIDES ENSSENTIAL FOR
       HUMAN BINDING

<220>
<221>  VARIANT
<222>  (7)..(7)
<223>  Xaa is any amino acid

<220>
<221>  VARIANT
<222>  (9)..(9)
<223>  Xaa is any amino acid

<220>
<221>  VARIANT
<222>  (21)..(22)
<223>  Xaa is any amino acid

<220>
<221>  VARIANT
<222>  (24)..(24)
<223>  Xaa is any amino acid

<220>
<221>  VARIANT
<222>  (29)..(29)
<223>  Xaa is any amino acid

<220>
<221>  VARIANT
<222>  (33)..(33)
<223>  Xaa is any amino acid

<220>
<221>  VARIANT

<222>  (40)..(40)
<223>  Xaa is any amino acid

<220>
<221>  VARIANT
<222>  (46)..(46)
<223>  Xaa is any amino acid

<400>  19

Met Val Lys Val Lys Phe Xaa Ile Xaa Gly Glu Glu Lys Glu Val Asp
1               5                   10                  15

Thr Ser Lys Ile Xaa Xaa Val Xaa Arg Ile Gly Lys Xaa Val Leu Phe
            20                  25                  30

Xaa Tyr Asp Asp Asn Gly Lys Xaa Gly Met Gly Leu Val Xaa Glu Lys
        35                  40                  45

Asp Ala Pro Lys Glu Leu Leu Asp Met Leu Ala Arg Ala Glu Arg Glu
    50                  55                  60

Lys Lys
65

<210>  20
<211>  66
<212>  PRT
<213>  Artificial Sequence

<220>
<223>  SAC7D VARIANT BINDING TO PD-L1 WITH AMINO ACIDES ENSSENTIAL FOR
       MOUSE PROTEIN BINDING

<220>
<221>  VARIANT
<222>  (7)..(7)
<223>  Xaa is any amino acid

<220>
<221>  VARIANT
<222>  (9)..(9)
<223>  Xaa is any amino acid

<220>
<221>  VARIANT
<222>  (21)..(22)
<223>  Xaa is any amino acid

<220>
<221>  VARIANT
<222>  (40)..(40)
<223>  Xaa is any amino acid

<220>
<221>  VARIANT
<222>  (46)..(46)

<223>  Xaa is any amino acid

<400>  20

Met Val Lys Val Lys Phe Xaa Ile Xaa Gly Glu Glu Lys Glu Val Asp
1               5                   10                  15

Thr Ser Lys Ile Xaa Xaa Val Arg Arg Ile Gly Lys Tyr Val Leu Phe
            20                  25                  30

Lys Tyr Asp Asp Asn Gly Lys Xaa Gly Met Gly Leu Val Xaa Glu Lys
        35                  40                  45

Asp Ala Pro Lys Glu Leu Leu Asp Met Leu Ala Arg Ala Glu Arg Glu
    50                  55                  60

Lys Lys
65

<210>  21
<211>  66
<212>  PRT
<213>  Artificial Sequence

<220>
<223>  SAC7D VARIANT BINDING TO PD-L1

<400>  21

Met Val Lys Val Lys Phe Val Ile Gly Gly Glu Glu Lys Glu Val Asp
1               5                   10                  15

Thr Ser Lys Ile Lys Thr Val Arg Arg Ile Gly Lys Tyr Val Leu Phe
            20                  25                  30

Lys Tyr Asp Asp Asn Gly Lys Thr Gly Met Gly Leu Val Ser Glu Lys
        35                  40                  45

Asp Ala Pro Lys Glu Leu Leu Asp Met Leu Ala Arg Ala Glu Arg Glu
    50                  55                  60

Lys Lys
65

<210>  22
<211>  64
<212>  PRT
<213>  Artificial Sequence

<220>
<223>  SSO7D VARIANT BINDING TO PD-L1 WITH AMINO ACIDES ENSSENTIAL FOR
       HUMAN PROTEIN BINDING

<220>
<221>  VARIANT
<222>  (7)..(7)
<223>  Xaa is any amino acid

<220>
<221>  VARIANT
<222>  (9)..(9)
<223>  Xaa is any amino acid

<220>
<221>  VARIANT
<222>  (21)..(22)
<223>  Xaa is any amino acid

<220>
<221>  VARIANT
<222>  (24)..(24)
<223>  Xaa is any amino acid

<220>
<221>  VARIANT
<222>  (29)..(29)
<223>  Xaa is any amino acid

<220>
<221>  VARIANT
<222>  (33)..(33)
<223>  Xaa is any amino acid

<220>
<221>  VARIANT
<222>  (41)..(41)
<223>  Xaa is any amino acid

<220>
<221>  VARIANT
<222>  (47)..(47)
<223>  Xaa is any amino acid

<400>  22

Met Ala Thr Val Lys Phe Xaa Ile Xaa Gly Glu Glu Lys Glu Val Asp
1               5                   10                  15

Ile Ser Lys Ile Xaa Xaa Val Xaa Arg Ile Gly Lys Xaa Ile Leu Phe
            20                  25                  30

Xaa Tyr Asp Glu Gly Gly Gly Lys Xaa Gly Met Gly Leu Val Xaa Glu
        35                  40                  45

Lys Asp Ala Pro Lys Glu Leu Leu Gln Met Leu Glu Lys Gln Lys Lys
    50                  55                  60

<210>  23
<211>  64
<212>  PRT
<213>  Artificial Sequence

<220>
<223>  SSO7D VARIANT BINDING TO PD-L1

<400>  23

Met Ala Thr Val Lys Phe Val Ile Gly Gly Glu Glu Lys Glu Val Asp
1               5                   10                  15

Ile Ser Lys Ile Lys Thr Val Arg Arg Ile Gly Lys Tyr Ile Leu Phe
            20                  25                  30

Lys Tyr Asp Glu Gly Gly Gly Lys Thr Gly Met Gly Leu Val Ser Glu
        35                  40                  45

Lys Asp Ala Pro Lys Glu Leu Leu Gln Met Leu Glu Lys Gln Lys Lys
    50                  55                  60

<210>  24
<211>  68
<212>  PRT
<213>  Artificial Sequence

<220>
<223>  CONSENSUS HSP110 BINDER WITH ESSENTIAL AMINO ACIDS

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (8)..(8)
<223>  Xaa is any amino acid

<220>
<221>  VARIANT
<222>  (15)..(15)
<223>  Xaa is E or Q

<220>
<221>  VARIANT

<222>  (18)..(18)
<223>  Xaa is T or I

<220>
<221>  misc_feature
<222>  (27)..(27)
<223>  Xaa can be any naturally occurring amino acid

<220>
<221>  VARIANT
<222>  (28)..(28)
<223>  Xaa is any amino acid

<220>
<221>  VARIANT
<222>  (30)..(30)
<223>  Xaa is any amino acid

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT
<222>  (44)..(44)
<223>  Xaa is any amino acid

<220>
<221>  VARIANT
<222>  (48)..(48)
<223>  Xaa is any amino acid

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(67)
<223>  Xaa is -------, EKS--GK, EK---QK, ARAEREK, ARA-EKK, E-----K,
       EKS--G, ACAEREK or ACA-EKK

<400>  24

Met Xaa Xaa Xaa Val Xaa Phe Xaa Trp Arg Gly Glu Glu Lys Xaa Val
1               5                   10                  15

Asp Xaa Ser Lys Ile Trp Asp Val Trp Arg Xaa Gly Lys Xaa Xaa Tyr
            20                  25                  30

Phe His Tyr Asp Xaa Xaa Xaa Gly Lys Met Gly Xaa Gly Lys Val Xaa
        35                  40                  45

Glu Lys Asp Ala Pro Lys Glu Leu Xaa Xaa Xaa Leu Xaa Xaa Xaa Xaa
    50                  55                  60

Xaa Xaa Xaa Lys
65

<210>  25
<211>  68
<212>  PRT
<213>  Artificial Sequence

<220>
<223>  CONSENSUS HSP110 BINDER

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (15)..(15)
<223>  Xaa is E or Q

<220>
<221>  VARIANT
<222>  (18)..(18)
<223>  Xaa is T or I

<220>
<221>  VARIANT
<222>  (31)..(31)
<223>  Xaa is V or I

<220>

<221>  VARIANT
<222>  (37)..(39)
<223>  Xaa Xaa Xaa is EGG or DN-

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(67)
<223>  Xaa is -------, EKS--GK, EK---QK, ARAEREK, ARA-EKK, E-----K,
      EKS--G, ACAEREK or ACA-EKK

<400>  25

Met Xaa Xaa Xaa Val Xaa Phe Met Trp Arg Gly Glu Glu Lys Xaa Val
1               5                   10                  15

Asp Xaa Ser Lys Ile Trp Asp Val Trp Arg Ser Gly Lys Thr Xaa Tyr
            20                  25                  30

Phe His Tyr Asp Xaa Xaa Xaa Gly Lys Met Gly Gln Gly Lys Val His
        35                  40                  45

Glu Lys Asp Ala Pro Lys Glu Leu Xaa Xaa Xaa Leu Xaa Xaa Xaa Xaa
    50                  55                  60

Xaa Xaa Xaa Lys
65

<210>  26
<211>  66
<212>  PRT
<213>  Artificial Sequence

<220>
<223>  SAC7D VARIANT BINDING TO HSP110 WITH ESSENTIAL AMINO ACIDS

<220>
<221>  VARIANT
<222>  (7)..(7)
<223>  Xaa is any amino acid

<220>
<221>  VARIANT

<222>  (26)..(26)
<223>  Xaa is any amino acid

<220>
<221>  VARIANT
<222>  (29)..(29)
<223>  Xaa is any amino acid

<220>
<221>  VARIANT
<222>  (42)..(42)
<223>  Xaa is any amino acid

<220>
<221>  VARIANT
<222>  (46)..(46)
<223>  Xaa is any amino acid

<400>  26

Met Val Lys Val Lys Phe Xaa Trp Arg Gly Glu Glu Lys Glu Val Asp
1               5                   10                  15

Thr Ser Lys Ile Trp Asp Val Trp Arg Xaa Gly Lys Xaa Val Tyr Phe
            20                  25                  30

His Tyr Asp Asp Asn Gly Lys Met Gly Xaa Gly Lys Val Xaa Glu Lys
        35                  40                  45

Asp Ala Pro Lys Glu Leu Leu Asp Met Leu Ala Arg Ala Glu Arg Glu
    50                  55                  60

Lys Lys
65

<210>  27
<211>  66
<212>  PRT
<213>  Artificial Sequence

<220>
<223>  SAC7D VARIANT BINDING TO HSP110

<400>  27

Met Val Lys Val Lys Phe Met Trp Arg Gly Glu Glu Lys Glu Val Asp
1               5                   10                  15

Thr Ser Lys Ile Trp Asp Val Trp Arg Ser Gly Lys Thr Val Tyr Phe
            20                  25                  30

His Tyr Asp Asp Asn Gly Lys Met Gly Gln Gly Lys Val His Glu Lys
        35                  40                  45

Asp Ala Pro Lys Glu Leu Leu Asp Met Leu Ala Arg Ala Glu Arg Glu

50                  55                  60

Lys Lys
65

<210>  28
<211>  64
<212>  PRT
<213>  Artificial Sequence

<220>
<223>  SSO7D VARIANT BINDING TO HSP110 WITH ESSENTIAL AMINO ACIDS

<220>
<221>  VARIANT
<222>  (7)..(7)
<223>  Xaa is any amino acid

<220>
<221>  VARIANT
<222>  (26)..(26)
<223>  Xaa is any amino acid

<220>
<221>  VARIANT
<222>  (29)..(29)
<223>  Xaa is any amino acid

<220>
<221>  VARIANT
<222>  (43)..(43)
<223>  Xaa is any amino acid

<220>
<221>  VARIANT
<222>  (47)..(47)
<223>  Xaa is any amino acid

<400>  28

Met Ala Thr Val Lys Phe Xaa Trp Arg Gly Glu Glu Lys Glu Val Asp
1               5                   10                  15

Ile Ser Lys Ile Trp Asp Val Trp Arg Xaa Gly Lys Xaa Ile Tyr Phe
            20                  25                  30

His Tyr Asp Glu Gly Gly Gly Lys Met Gly Xaa Gly Lys Val Xaa Glu
        35                  40                  45

Lys Asp Ala Pro Lys Glu Leu Leu Gln Met Leu Glu Lys Gln Lys Lys
    50                  55                  60

<210>  29
<211>  64
<212>  PRT
<213>  Artificial Sequence

<220>
<223>  SSO7D VARIANT BINDING TO HSP110

<400>  29

Met Ala Thr Val Lys Phe Met Trp Arg Gly Glu Glu Lys Glu Val Asp
1               5                   10                  15

Ile Ser Lys Ile Trp Asp Val Trp Arg Ser Gly Lys Thr Ile Tyr Phe
            20                  25                  30

His Tyr Asp Glu Gly Gly Gly Lys Met Gly Gln Gly Lys Val His Glu
        35                  40                  45

Lys Asp Ala Pro Lys Glu Leu Leu Gln Met Leu Glu Lys Gln Lys Lys
    50                  55                  60

## Description

The invention is in the field of cancer treatment and relates in particular to the development of new binders to PDL-1 usable for such purpose.

### Introduction

Cancer immunotherapy has become a treatment of choice for some cancers. It consists essentially in using the patient's immune system to fight the cancer cells. In particular, cell-based immunotherapies make use of immune effector cells such as lymphocytes (notably T lymphocytes) as well as of non-specialized cells such as macrophages, dendritic cells, or natural killer cells (NK Cell) to target tumor antigens expressed on the surface of tumor cells.

It has however been observed that tumor cells are able to evade the immune response by stimulating immune checkpoint targets. Immune checkpoints are regulators of the immune system can lower of inhibit the immune response when stimulated.

Immune checkpoints include CTLA4, PD-1, and PD-L1. PD-1 is the transmembrane programmed cell death 1 protein (also called PDCD1 and CD279), and interacts with PD-L1 (PD-1 ligand 1, or CD274). Upregulation of PD-L1 on the cell surface may inhibit T cells. Blocking the interaction between PD-1 and PD-L1 may allow the T-cells to fight the tumor cells. This protein is present in the UNiprot database under accession number Q9NZQ7 (human protein) or Q9EP73 (mouse protein). RefSeq accession numbers are NP_001254635, NP_001300958 and NP_054862 (human protein) and NP_068693 (mouse protein).

HSP110, also called HSPH1 or Hsp105 (Hsp105a or Hsp105β), is a mammalian member of the HSP105/110 family, a subgroup of the HSP70 family. Hsp105a is expressed constitutively and in response to various forms of stress, while Hsp105β is an alternatively spliced form of Hsp105α that is expressed specifically during mild heat shock and was cloned by Yasuda et al (J Biol Chem 270, 29718-29723) and Ishihara et al (Biochim Biophys Acta 1444, 138-142). HSP110 has been shown as being a major determinant for both prognosis and treatment response in colorectal cancer (CRC), in particular by Dorard et al (Nat Med. 2011 Sep 25;17(10):1283-9). In particular, the binding between Hsp110 and STAT3 allows controlling cancers, notably CRC cancers (Gozzi et al, Cell Death Differ. 2020 Jan;27(1):117-129), and may improve response to chemotherapies (Dubrez et al, Oncogene. 2020 Jan;39(3):516-529). This protein is present in the UNiprot database under accession number Q92598 (human protein) or Q61699 (mouse protein). It is thus interesting to identify inhibitors of the HSP110-STAT3 binding.

The epidermal growth factor receptor (EGFR; ErbB-1; HER1 in humans) is a transmembrane protein that is a receptor for members of the epidermal growth factor family (EGF family) of extracellular protein ligands. EGFR overexpression has been associated with a number of cancers, including adenocarcinoma of the lung (40% of cases), anal cancers, glioblastoma (50%) and epithelian tumors of the head and neck (80-100%). Mutations, amplifications or misregulations of EGFR or family members are implicated in about 30% of all epithelial cancers. Anticancer drugs targeting EGFR have been developed, including gefitinib, erlotinib, afatinib, brigatinib and icotinib for lung cancer, and cetuximab for colon cancer. Other drugs include panitumumab and osimertinib. Such of these drugs are monoclonal antibodies, whereas others are tyrosine kinase inhibitors. This protein is present in the UNiprot database under accession number P00533 (human protein) or Q01279 (mouse protein).

### SUMMARY OF THE INVENTION

The invention relates a polypeptide comprising a variant of the Sac7d protein or of a protein of the Sac7d family that binds to the PD-L1. Such variant is able to be used for cancer treatment, as it makes it possible to decrease tumor weight, as shown in the examples. Such variant comprises SEQ ID NO: 16, SEQ ID NO: 17 or SEQ ID NO: 18. In particular, said variant is a variant of the Sac7d protein, and comprises SEQ ID NO: 19, SEQ ID NO: 20 or SEQ ID NO: 21. In another embodiment, said variant is a variant of the Sso7d protein, and comprises SEQ ID NO: 22 or SEQ ID NO: 23.

The invention also relates to a polypeptide comprising a variant of the Sac7d protein or of a protein of the Sac7d family that binds to the HSP110. Such variant is able to be used for cancer treatment, as it makes it possible to decrease tumor weight, as shown in the examples. Such variant comprises SEQ ID NO: 24 or SEQ ID NO: 25. In particular, said variant is a variant of the Sac7d protein, and comprises SEQ ID NO: 26 or SEQ ID NO: 27. In another embodiment, said variant is a variant of the Sso7d protein, and comprises SEQ ID NO: 28 or SEQ ID NO: 29. Such variant inhibits the binding of HSP110 to Stat3.

It is to be noted that the sequences indicated above comprise a methionine at its N-terminal, but that it is also possible to perform the invention when such methionine listed at the N-terminal end of each sequence is not included.

It is to be noted that the specification discloses variants of Sac7d, but the teachings are applicable to the other proteins of the Sac7d family. The teachings are also applicable to other OB-fold domains as disclosed in WO2007139397. The invention is also applicable to SH3 domains, a small protein domain of about 60 amino acid residues, initially, described as a conserved sequence in the viral adaptor protein v-Crk and described under PF00018 in the PFAM database. The SH3 domain has a characteristic beta-barrel fold that consists of five or six β-strands arranged as two tightly packed anti-parallel β sheets. The linker regions may contain short helices. It is to be noted that OB-fold and SH3 domains share homology and that, in view of the knowledge of the sequence and structure of these domains, it is possible to determine, in any of OB-fold or SH3 domain, to which amino acids correspond the amino acids as disclosed below for Sac7d.

In a specific embodiment, the polypeptide consists in the variant of a protein of the Sac7d family binding to PD-L1. In a specific embodiment, the polypeptide consists in the variant of a protein of the Sac7d family binding to HSP110.

In a specific embodiment, the variant of a protein of the Sac7d family binding to PD-L1 is linked or fused to another protein or polypeptide. In particular, the other protein or polypeptide comprises another variant of a protein of the Sac7d family. In this embodiment, it is preferred when the other variant of the Sac7d family binds to HSP110. Such other variant may comprise SEQ ID NO: 24, SEQ ID NO: 25, SEQ ID NO: 26, SEQ ID NO: 27, SEQ ID NO: 28 or SEQ ID NO: 29. SEQ ID NO: 26 or SEQ ID NO: 27 are particularly preferred. In another embodiment, the other variant of the Sac7d family binds to EGFR.

In specific embodiments, the variant is present in a polypeptide and is thus covalently linked by amine bounds to other proteins presenting a biological interest.

In an embodiment, the polypeptide is conjugated to an organic molecule that presents some functionality, in particular a tyrosine kinase inhibitor that is used as a EGFR inhibitor.

The invention also pertains to a genetic construct comprising a DNA sequence coding for the polypeptide described herein, to a vector comprising such genetic construct, and to a host cell comprising the genetic construct in its genome.

The invention also pertains to a method for producing the polypeptide herein disclosed, comprising the steps consisting of
a. Culturing a cell culture wherein the cells have been transformed by a genetic construct as disclosed,
   and
b. Recovering the polypeptide.

The invention also relates to a polypeptide herein disclosed as a medicament.

The invention also relates to a polypeptide herein disclosed for use thereof for the treatment of cancer, alone or in combination with chemotherapy or treatment with CAR-T cells.

The invention also relates to a method for treating a subject in need thereof comprising administering a therapeutic amount of a polypeptide herein disclosed to the subject, in particular when the subject has a cancer.

The invention also relates to a composition containing a polypeptide according to any one of claims 1 to 12 and a chemotherapy agent or CAR-T cells for simultaneous, separate or sequential (spread out over time) use in the treatment of cancer.

It is particularly adapted when the cancer is as disclosed below.

The invention also relates to these variants in therapeutic, diagnostic or purification uses. The invention also relates to composition, in particular oral or topical (dermal), containing the polypeptide or the variants.

It is reminded that the sequence of Sac7d is:

MVKVKFKYKGEEKEVDTSKIKKVWRVGKMVSFTYDDNGKTGRGAVSEKDAPKEL
LDMLARAEREKK (SEQ ID NO: 1).

The variants binding to PD-L1 herein disclosed contain mutations at positions corresponding to the positions 7, 8, 9, 21, 22, 24, 26, 29, 31, 33, 40, 42, 44 and 46 of the Sac7 sequence. It is however to be noted that the lysine at position 21 may be maintained in the variants, as well as the threonine at position 40, or the serine at position 46.

In preferred embodiments, the polypeptide comprises the sequence

MXXXVXFXIXGEEKXVDXSKIXXVXRIGKXXLFXYDXXXGKXGMGLVXEKDAPKEL
XXXLXXXXXXXK (SEQ ID NO: 16).

In this sequence, the amino acids designated as X at positions 8, 10, 22-23, 25, 30, 34, 42, 48 can be any amino acid. The other amino acids designated as X are indicated in table 1 (where "-" designates no amino acid).

**Table 1. description of amino acids**

| **Position** | **Amino acid** |
|---|---|
| 2 | X is V, A or T |
| 3 | X is T or K |
| 4 | X is - or K |
| 6 | X is R or K |
| 15 | X is E or Q |
| 18 | X is T or l |
| 31 | X is V or I |
| 37 to 39 | XXX is EGG or DN- |
| 57 | X is M or L |
| 58 | X is Q, D or E |
| 59 | X is M or K |
| 61 to 67 | As in the sequence listing |

In another embodiment, the polypeptide comprises the sequence

MXXXVXFXIXGEEKXVDXSKIXXVRRIGKYXLFKYDXXXGKXGMGLVXEKDAPKE
LXXXLXXXXXXXK (SEQ ID NO: 17).

In this sequence, the amino acids designated as X at positions 8, 10, 22-23, 42, 48 can be any amino acid. The other amino acids designated as X are indicated in table 1.

In another embodiment, the polypeptide comprises the sequence

MXXXVXFVIGGEEKXVDXSKIKTVRRIGKYXLFKYDXXXGKTGMGLVSEKDAPKE
LXXXLXXXXXXXK (SEQ ID NO: 18).

In this sequence, the amino acids designated as X are indicated in table 1.

In a preferred embodiment, the polypeptide comprises the sequence

MVKVKFXIXGEEKEVDTSKIXXVXRIGKXVLFXYDDNGKXGMGLVXEKDAPKELLD
MLARAEREKK (SEQ ID NO: 19).

In this sequence, the amino acids designated as X at positions 7, 9, 21-22, 24, 29, 33, 40, 46 can be any amino acid.

In a preferred embodiment, the polypeptide comprises the sequence

MVKVKFXIXGEEKEVDTSKIXXVRRIGKYVLFKYDDNGKXGMGLVXEKDAPKELL
DMLARAEREKK (SEQ ID NO: 20).

In this sequence, the amino acids designated as X at positions 7, 9, 21-22, 40, 46 can be any amino acid.

In a preferred embodiment, the polypeptide comprises the sequence MVKVKFVIGGEEKEVDTSKIKTVRRIGKYVLFKYDDNGKTGMGLVSEKDAPKELL DMLARAEREKK (SEQ ID NO: 21). Such sequence is particularly preferred.

In another embodiment, the polypeptide comprises the sequence MATVKFXIXGEEKEVDISKIXXVXRIGKXILFXYDEGGGKXGMGLVXEKDAPKELL QMLEKQKK (SEQ ID NO: 22). In this sequence, the amino acids designated as X at positions 7, 9, 21-22, 24, 29, 33, 41, 47 can be any amino acid.

In another embodiment, the polypeptide comprises the sequence

MATVKFVIGGEEKEVDISKIKTVRRIGKYILFKYDEGGGKTGMGLVSEKDAPKELL
QMLEKQKK (SEQ ID NO: 23).

The polypeptide may also comprise SEQ ID NO: 21, where between 1 to 10, more preferably from 1 to 8, more preferably from 1 to 6, more preferably from 1 to 5, more preferably from 1 to 4, more preferably from 1 to 3, more preferably 2, more preferably 1 amino acid selected from the group consisting of V7, G9, K21, T22, R24, Y29, K33, T40, and S46, more preferably from the group consisting of V7, G9, K21, T22, T40, and S46, have been replaced by any other amino acid.

The variants binding to HSP110 herein disclosed contain mutations at positions corresponding to the positions 7, 8, 9, 21, 22, 24, 26, 29, 31, 33, 40, 42, 44 and 46 of the Sac7 sequence. It is however to be noted that the tryptophan at position 24 may be maintained in the variants.

In preferred embodiments, the polypeptide comprises the sequence

MXXXVXFXWRGEEKXVDXSKIWDVWRXGKXXYFHYDXXXGKMGXGKVXEKDA
PKELXXXLXXXXXXXK (SEQ ID NO: 24).

In this sequence, the amino acids designated as X at positions 8, 28, 30, 44, 48 can be any amino acid. The other amino acids designated as X are indicated in table 1 (where "-" designates no amino acid).

In another embodiment, the polypeptide comprises the sequence

MXXXVXFMWRGEEKXVDXSKIWDVWRSGKTXYFHYDXXXGKMGQGKVHEKDA
PKELXXXLXXXXXXXK (SEQ ID NO: 25).

In this sequence, the amino acids designated as X are indicated in table 1.

In a preferred embodiment, the polypeptide comprises the sequence

MVKVKFXWRGEEKEVDTSKIWDVWRXGKXVYFHYDDNGKMGXGKVXEKDAPK
ELLDMLARAEREKK (SEQ ID NO: 26).

In this sequence, the amino acids designated as X at positions 7, 26, 29, 42, 46 can be any amino acid.

In a preferred embodiment, the polypeptide comprises the sequence MVKVKFMWRGEEKEVDTSKIVWRSGKTVYFHYDDNGKMGQGKVHEKDAPK ELLDMLARAEREKK (SEQ ID NO: 27). Such sequence is particularly preferred.

In another embodiment, the polypeptide comprises the sequence MATVKFXWRGEEKEVDISKIWDVWRXGKXIYFHYDEGGGKMGXGKVXEKDAPK ELLQMLEKQKK (SEQ ID NO: 28). In this sequence, the amino acids designated as X at positions 7, 26, 29, 43, 47 can be any amino acid.

In another embodiment, the polypeptide comprises the sequence

MATVKFMWRGEEKEVDISKIWDVWRSGKTIYFHYDEGGGKMGQGKVHEKDAPK
ELLQMLEKQKK (SEQ ID NO: 29).

The polypeptide may also comprise SEQ ID NO: 29, where between 1 to 5, more preferably from 1 to 4, more preferably from 1 to 3, more preferably 2, more preferably 1 amino acid selected from the group consisting of M7, S26, T29, Q43, and H47 have been replaced by any other amino acid.

In some embodiments, the polypeptide is such that the variant of a protein of the Sac7d family binding to PDL-1 is linked or fused to another protein or polypeptide, in particular another variant of a protein of the Sac7d family or an antibody, in particular binding to HSP-110 (in particular the ones disclosed by SEQ ID NO: 24 to SEQ ID NO 29, and in particular SEQ ID NO: 27) or to EGFR.

In some embodiments, the polypeptide is such that the variant of a protein of the Sac7d family binding to HSP-110 is linked or fused to another protein or polypeptide, in particular another variant of a protein of the Sac7d family or an antibody, in particular binding to PDL-1 (in particular the ones disclosed by SEQ ID NO: 16 to SEQ ID NO 23, and in particular SEQ ID NO: 21) or to EGFR.

In another embodiment, the polypeptide is conjugated to an organic molecule, in particular a tyrosine kinase inhibitor.

It also allows obtaining a genetic construct comprising a DNA sequence coding for a polypeptide defined above, a vector comprising such genetic construct and a host cell comprising such genetic construct in its genome.

It is possible to perform a method for producing the polypeptide, comprising culturing a cell culture wherein the cells have been transformed by a genetic construct, and recovering the polypeptide.

One can also embody a polypeptide as disclosed as a medicament.

The invention also pertains to a polypeptide herein disclosed, wherein said polypeptide binds to PDL-1, HSP-110 or EGFR, alone or in combination including as a multimeric polypeptide), for use thereof for the treatment of cancer, in particular solid tumors and lymphomas.

Such cancer is in particular non-small cell lung carcinoma, urothelial carcinoma, gastric cancer, liver cancer, kidney cancer, head and neck squamous cell cancer, esophageal squamous cell carcinoma, primary mediastinal B-cell lymphoma, classical Hodgkin lymphom, melanoma, merkel cell carcinoma, cervical cancer, microsatellite instability-high cancer, bladder cancer, breast cancer, small cell lung cancer, colorectal cancer, pancreatic cancer, prostate cancer.

The anti-PD-L1 variant is useful for a broad range of cancers as disclosed above and in particular for non-small cell lung carcinoma, urothelial carcinoma, gastric cancer, liver cancer, kidney cancer, head and neck squamous cell cancer, esophageal squamous cell carcinoma, primary mediastinal B-cell lymphoma, classical Hodgkin lymphom, melanoma, merkel cell carcinoma, cervical cancer, microsatellite instability-high cancer, bladder cancer, breast cancer, small cell lung cancer which are already treated with anti-PD-L1 agents.

The anti-HSP110 variant is particularly useful for treating colorectal cancer, pancreatic cancer, prostate cancer or breast cancer.

The invention also pertains to a polypeptide herein disclosed, wherein said polypeptide binds to PDL-1, HSP-110 and/or EGFR, for its use for the treatment of cancer in combination with chemotherapy of treatment with CAR-T cells, in particular the cancers cited above.

### DETAILED DESCRIPTION OF THE INVENTION

### The Sac7d protein family

The Sac7d family is defined as relating to the Sac7d protein and corresponds to a family of 7 kDa DNA-binding proteins isolated from extremophilic bacteria. It is herein disclosed as a representative species of OB-fold domains, that is preferably used in the context of the invention. Since SH3 domains share homology with OB-fold domains, the teachings pertaining to Sac7d are also applicable to SH3 scaffolds.

These proteins and this family are in particular described in WO 2008/068637. Thus, within the context of the present invention, a protein belongs to the Sac7d family when it has one of the sequences SEQ ID NO: 1 to SEQ ID NO: 14, or when it has a sequence corresponding to the sequence SEQ ID NO: 15, which is a consensus sequence (obtained from SEQ ID NO: 1 to SEQ ID NO: 9 and SEQ ID NO: 12 to SEQ ID NO: 14. In this consensus sequence, a dash - indicates no amino acid, the proteins not having all the same size). This Sac7d family comprises in particular the Sac7d or Sac7e proteins derived from *Sulfolobus acidocaldarius,* the Sso7d protein derived from *Sulfolobus solfataricus,* the DBP 7 also called Sto7 protein derived from *Sulfolobus tokodaii,* the Ssh7b protein derived from *Sulfolobus shibatae,* the Ssh7a protein derived from *Sulfolobus shibatae,* Mse7 derived from *Metallosphaera sedula,* Mcu7 derived from *Metallosphaera cuprina,* Aho7a or Aho7b or Aho7c derived from *Acidianus hospitalis,* Sis7a or Sis7b derived from *Sulfolobus islandicus* and the p7ss protein derived from *Sulfolobus solfataricus.* In view of the broad similarity of the sequences of the proteins of the Sac7d family, it is directly possible and easy to identify the amino acids of another protein than Sac7d that correspond to given amino acids of Sac7d. In particular, one can also use the teachings of WO 2008/068637 that shows (in the figures) and explains (in the specification) that such proteins are superimposable.

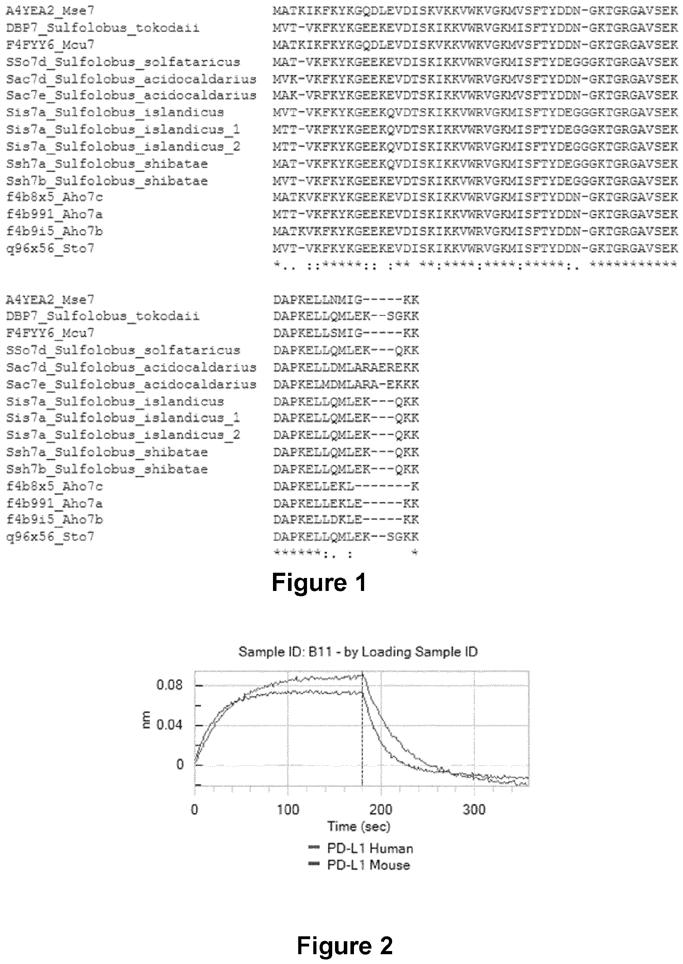

WO 2012/150314 shows that the mutations from one protein of the Sac7d family can be carried to another protein of the same family. This portability amounts to creating a mutant of another protein of the Sac7d family, starting from a mutant of one protein of said family. The first mutant may have been obtained in particular by carrying out the process of WO 2008/068637. Consequently, it is possible, using in particular the teachings of WO 2012/150314 and of figure 1, to obtain a mutant of any protein of the Sac7d family, starting from a mutant of any other protein of such family. As an illustration, for a mutant of Sac7d, one can introduce the mutated amino acids of the Sac7d mutant within the scaffold of another protein, using the sequence alignment of figure 1. As an illustration, variants of Sso7d, obtained from the Sac7d variants herein disclosed, are described as SEQ ID NO: 22, SEQ ID NO: 27, SEQ ID NO: 28 and SEQ ID NO: 29.

The number of mutated residues introduced within a wild-type protein sequence to obtain a variant is preferably between 4 and 25, or more specifically between 4 and 22. It is thus possible to obtain variants preferably having at least 4, more preferably at least 5, more preferably at least 6, more preferably at least 7 or 8, even more preferably at least 10, but generally less than 25, more preferably less than 22, even more preferably less than 20 or less than 15 or 14 substituted amino acids compared with the wild-type OB-fold protein (or domain). It is to be noted that all and any ranges are considered herein, (such as 5-20 or 7-25 and so forth). Particularly preferred ranges are 4-17 and 6-17, 4-14 and 6-14.

It is preferred when 7, 8, 9, 10, 11, 12, 13 or 14 amino acids are mutated in the binding site of the OB-fold domain, relative to the wild-type OB-fold domain. These mutations are introduced in the amino acids corresponding to V2, K3, K5, K7, Y8, K9, G10, E11, K13, E14, T17, K21, K22, W24, V26, G27, K28, M29, S31, T33, Y34, D36, N37, G38, K39, T40, R42, A44, S46, E47, K48, D49, A50 and P51 of Sac7d (SEQ ID NO: 1)

In particular, it is interesting when the number of mutated amino acids is between 7 and 14 (limits included). In particular, the variants can also comprise amino acid insertions as indicated above.

As indicated, proteins of the Sac7d family are Sac7d or Sac7e derived from *Sulfolobus acidocaldarius,* Sso7d derived from *Sulfolobus solfataricus,* DBP 7 also called Sto7 derived from *Sulfolobus tokodaii,* Ssh7b derived from *Sulfolobus shibatae,* Ssh7a derived from *Sulfolobus shibatae,* Mse7 derived from *Metallosphaera sedula,* Mcu7 derived from *Metallosphaera cuprina,* Aho7a or Aho7b or Aho7c derived from *Acidianus hospitalis,* Sis7a or Sis7b derived from *Sulfolobus islandicus* and p7ss derived from *Sulfolobus solfataricus.* The various sequences of the Sac7d, Sso7d, Sac7e, Ssh7b, Ssh7a, DBP7, Sis7a (3 alleles), Mse7, Mcu7, Aho7a, Aho7b and Aho7c proteins are represented by SEQ ID NO: 1 to SEQ ID NO: 14 respectively.

A variant of a protein of this Sac7d family may be called a nanofitin. The invention is thus preferentially implemented on variants of the proteins represented by SEQ ID NO: 1 to SEQ ID NO: 14, or a protein having the sequence SEQ ID NO: 15, in particular on variants of Sac7d.

### Link of Sac7d protein with OB-fold proteins and SH3 domains

The OB-fold proteins are known in the art. They are in particular described in the documents cited above, and also in Arcus (Curr Opin Struct Biol. 2002 Dec; 12(6):794-801). OB-fold is in the form of a cylinder having five beta (β) sheets. Most OB-fold proteins use the same binding interface of their natural ligand, which may be an oligosaccharide, an oligonucleotide, a protein, a metal ion or a catalytic substrate. This binding interface comprises mainly the residues located in the beta sheets. Certain residues located in the loops may also be involved in the binding of an OB-fold protein with its natural ligand. Thus, applications WO 2007/139397 and WO 2008/068637 and the Arcus document (2002, op. cit.) describe the OB-fold-protein domains for binding with their natural ligand.

In particular, document WO 2008/068637 describes precisely how to identify the binding domain of an OB-fold protein. By superimposing several sequences and 3D structures of proteins having OB-fold domains, using the websites WU-Blast2 (http://www.ebi.ac.uk/blast2/index.html) (Lopez et al., 2003, Nucleic Acids Res 31, 3795-3798), T-COFFEE (http://www.ch.embnet.org/software/TCoffee.html) (Notredame et al., 2000, J Mol Biol 302, 205-217) and DALI lite (http://www.ebi.ac.uk/DaliLite/) (Holm and Park, 2000, Bioinformatics 16, 566-567), it is possible to identify the positions of the binding domains and in particular the amino acids which can be modified. Taking the sequence of Sac7d (SEQ ID NO: 1) as a reference, these are the residues V2, K3, K5, K7, Y8, K9, G10, E11, K13, E14, T17, K21, K22, W24, V26, G27, K28, M29, S31, T33, Y34, D35, D36, N37, G38, K39, T40, G41, R42, A44, S46, E47, K48, D49, A50 and P51.

WO 2008/068637 describes that it is possible to perform a superimposition of 3D structures of OB-fold proteins or domains (10 domains were used in this application, including Sac7d), using the DALI website (http://www.ebi.ac.uk/dali/interactive.html)(Holm and Sander, 1998, Nucleic Acids Res 26, 316-319). Thus, it is easy to identify, for any OB-fold protein (or any OB-fold domain), the amino acids involved in the binding site and corresponding to the Sac7d amino acids mentioned above. Consequently, providing the amino acids that can be mutated in one of these proteins makes it possible to identify the corresponding amino acids for any other OB-fold domain.

It is also to be noted that OB-fold domains resemble SH3 domains and that it is also possible to identify equivalents of amino acids of Sac7d in SH3 domains.

### Example of OB-fold or SH3 domain

Non-limitative examples of OB-fold proteins which can be used according to the invention are Sac7d, Sso7d, the N-terminal domain of SEB (Papageorgiou et al., 1998), the chain A of the Shiga-like toxin Ile (PDB 2bosa), the human Neutrophil Activatin Peptide-2 (NAP-2, PDB 1tvxA), the Molybdenum Binding Protein (modg) of Azotobacter vinelandii (PDB 1h9j), the N-terminal domain of SPE-C (Roussel et al., 1997), the B5 subunit of E. coli Shiga-like toxin (Kitov et al., 2000), Cdc13 (Mitton-Fry et al., 2002), the cold-shock DNA-binding domain of the human Y-box protein YB-1 (Kloks et al., 2002), the E. coli inorganic pyrophosphatase EPPase (Samygina et al., 2001), or any of the proteins listed in Table 3 of the article by (Arcus, 2002), such as 1krs (Lysyl-tRNA synthetase LysS, E.coli), 1c0aA (Asp- tRNA synthetase, E.coli), 1b8aA (Asp- tRNA synthetase, P. kodakaraensis), 1lylA (Lysyl-tRNA synthetase LysU, E.coli), 1quqA (Replication protein A, 32kDa subunit, Human), 1quqB (Replication protein A, 14kDa subunit, Human), 1jmcA (Replication protein A, 70kDa subunit (RPA70) fragment, Human), 1otc (Telomere-end-binding protein, O. nova), 3ullA (Mitochondrial ssDNA-binding protein, Human), 1prtF (Pertussis toxin S5 subunit, B. pertussis), 1bcpD (Pertussis toxin S5 subunit (ATP bound), B. pertussis), 3chbD (Cholera Toxin, V. cholerae), 1tiiD (Heat-labile toxin, E. coli), 2bosA (Verotoxin-1/Shiga toxin, B-pentamer, E. coli), 1br9 (TIMP-2, Human), 1an8 (Superantigen SPE-C, S. pyogenes), 3seb (Superantigen SPE, S. aureus), 1aw7A (Toxic shock syndrome toxin, S. aureus), 1jmc (Major cold-shock protein, E.coli), 1bkb (Initiation translation factor 5a, P. aerophylum), 1sro (S1 RNA-binding domain of PNPase, E.coli), 1d7qA (Initiation translation factor 1, elF1a, Human), 1ah9 (Initiation translation factor 1, IF1, E.coli), 1b9mA (Mo-dependent transcriptional regulator ModE, E.coli), 1ckmA (RNA guanylyltransferase, Chlorella virus, PBCV-1), 1a0i (ATP-dependent DNA ligase, Bacteriophage T7), 1snc (Staphylococcal nuclease, S. aureus),1hjp (DNA helicase RuvA subunit, N-terminal domain, E.coli), 1pfsA (Gene V protein, Pseudomonas bacteriophage pf3), 1gvp (Gene V protein, Filamentous bacteriophage (f1, M13)), 1gpc (Gene 32 protein (gp32) core, Bacteriophage T4), 1wgjA (Inorganic pyrophosphatase, S. cerevisiae), and 2prd (Inorganic pyrophosphatase, T. thermophilus).

Non-exhaustive examples of proteins with SH3 domaines are Signal transducing adaptor proteins, CDC24, Cdc25, Pl3 kinase, Phospholipase, Ras GTPase-activating protein, Vav proto-oncogene, GRB2, p54 S6 kinase 2 (S6K2), SH3D21, ClOorf76 (potentially), STAC3, Some myosins, SHANK1,2,3, ARHGAP12, C8orf46, TANGO1, Integrase, Focal Adhesion Kinase (FAK, PTK2), Proline-rich tyrosine kinase (Pyk2, CADTK, PTK2beta), or TRIP10 (cip4).

### Production of the identified variants

The sequence of the identified variant can be cloned in any appropriate vector by any molecular genetic methods known in the art.

These recombinant DNA constructs comprising a nucleotide sequence, coding for a polypeptide comprising a variant as described above, are used in connection with a vector, such as a plasmid, phagemid, phage or viral vector.

These recombinant acid molecules can be produced by techniques described in Sambrook et al., 1989 (Sambrook J, Fritschi EF and Maniatis T (1989) Molecular cloning: a laboratorymanual, Cold Spring Harbor Laboratory Press, New York). Alternatively, the DNA sequences may be chemically synthesized using, for example, synthesizers.

Recombinant constructs of the invention comprise the expression vectors that are capable of expressing the RNA and thus lead to production of proteins from the above genetic sequences. The vector may thus further comprise regulatory sequences, including a suitable promoter operably linked to the open reading frame (ORF) of the genetic sequences herein disclosed. The vector may further comprise a selectable marker sequence such as an antibiotic resistance gene. Specific initiation and bacterial secretory signals also may be required for efficient translation of the coding sequences when bacteria as used as the expression host.

### Production of the molecules

Cells are transfected or transformed with vectors containing the sequences coding for the polypeptides comprising the variant as disclosed above.

The cells are the cultured in such conditions as to have the protein expressed and favorably secreted. The conditions of culture of the cells are the conditions generally used for recombinant antibody production and are known in the art. Such conditions that are known in the art can also be optimized by the person skilled in the art if needed. Kunert and Reinhart (Appl Microbiol Biotechnol. 2016; 100: 3451-3461) review such methods and provide ample references thereto.

One can use bacterial, phage (Shukra et al, Eur J Microbiol Immunol (Bp). 2014; 4(2): 91-98) or eukaryotic systems of production.

One shall prefer to use eukaryotic cells in order to obtain proper post-translational modifications such as glycosylation.

In particular, one can use CHO (Chinese Hamster Ovary) cells, PER.C6 cells (human cell line, Pau et al, Vaccine. 2001 21;19(17-19):2716-21), HEK 293b cells (Human embryonic kidney cells 293), NSO cells (cell line derived from the non-secreting murine myeloma) or EB66 cells (a duck cell line Valneva, Lyons, France).

Also provided by the present disclosure are host cells containing at least one of the DNAs constructs coding for a polypeptide comprising the variant as disclosed herein. The host cell can be any cell for which expression vectors are available. As indicated above, it may be a higher eukaryotic host cell, such as a mammalian cell, a lower eukaryotic host cell, such as a yeast cell, or a prokaryotic cell, such as a bacterial cell.

Introduction of the recombinant construct into the host cell is performed by any method known in the art (such as calcium phosphate transfection, lipofection, DEAE, dextran mediated transfection, electroporation or phage infection). The vectors can be inserted within the genome of the host cell, or be maintained as an extragenomic vector (such as a Bacterial Artificial Chromosome or a Yeast Artificial Chromosome). When introduced within the cell genome, such introduction may be random or targeted using methods known in the art (homologous recombination or the like).

### Bacterial hosts and expression

Useful expression vectors for bacterial use are constructed by inserting the recombinant DNA sequence together with suitable translation initiation and termination signals in operable reading phase with a functional promoter. The vector will comprise one or more phenotypic selectable markers and an origin of replication to ensure maintenance of the vector and, if desirable, to provide amplification within the host.

Suitable prokaryotic hosts for transformation include *E. coli, Bacillus subtilis, Salmonella typhimurium* and various species within the genera *Pseudomonas, Streptomyces,* and *Staphylococcus.*

### Eukaryotic hosts and expression

Examples of the eukaryotic host cells include vertebrate cells, insect cells, and yeast cells. In particular, one can use the cells mentioned above.

The transformed or transfected cells are cultured according to methods known in the art and the polypeptide are recovered from intracellular or extracellular fractions (depending on whether it is secreted or not).

### Isolation of the molecules

The recombinant protein produced can be separated and purified by any of various known separation methods utilizing the physical or chemical property of the protein, from the intracellular or extracellular fraction.

In particular, one can use methods such as precipitation, ultrafiltration, various types of liquid chromatography such as molecular sieve chromatography (gel filtration), adsorption chromatography, ion exchange chromatography, and affinity chromatography, dialysis, and a combination thereof.

In general, any method known and used to purify recombinant polypeptides is adapted for the purification of the molecules herein disclosed.

If a tag has been introduced within the recombinant sequence (such as a poly-Histidine tag), one can purify the molecules using this tag. However, it is preferred to use affinity to purify the molecules.

One can, in particular, use the fact that the molecule herein produced binds to a specific target and use any affinity method (affinity column, FACS, beads) to isolate such molecules.

One particular advantage of the molecules herein disclosed is that they don't need to be glycosylated to be active and can thus be produced in any type of cells, and not necessarily eukaryotic cells. They are particularly well produced in bacterial cells.

### Modification of the variants

The variants described above, and having the ability to bind PD-L1 or HSP110 can be modified by any method known in the art.

### Preparing a polypeptide comprising the variant

It is possible to prepare a DNA sequence that would contain two coding sequences, one for the variant herein disclosed and another for a protein or peptide of interest. The resulting expressed protein would thus be a polypeptide that would contain both proteins. The vector may be built in such a way that it would contain a sequence coding for a linker that would be located between the two proteins in the expressed polypeptide.

Consequently, the invention also encompasses a polypeptide comprising the variant of a protein of the OB-fold protein (preferably of the Sac7d family) binding to PDL-1, or HSP110 which is linked (preferably through amine bound as disclosed above) to another protein or polypeptide.

In a specific embodiment, the other protein or polypeptide comprises another variant of a protein of the Sac7d family, in particular as herein disclosed.

Are of particular interest
- A polypeptide comprising a variant of an OB-fold protein of the Sac7d family binding to HSP-110, fused with a variant of an OB-fold protein of the Sac7d family binding to PD-L1 (in N- or C-terminus)
- A polypeptide comprising a variant of an OB-fold protein of the Sac7d family binding to HSP-110, fused with a variant of an OB-fold protein of the Sac7d family binding to EGFR (in N- or C-terminus)
- A polypeptide comprising a variant of an OB-fold protein of the Sac7d family binding to HSP-110, fused with the same or another variant of an OB-fold protein of the Sac7d family binding to HSP110 (in N- or C-terminus)
- A polypeptide comprising a variant of an OB-fold protein of the Sac7d family binding to PD-L1, fused with the same or another variant of an OB-fold protein of the Sac7d family binding to PD-L1 (in N- or C-terminus)
- A polypeptide comprising a variant of an OB-fold protein of the Sac7d family binding to PD-L1, fused with a variant of an OB-fold protein of the Sac7d family binding to EGFR (in N- or C-terminus).

In another embodiment, the other protein or polypeptide is an antibody. In this embodiment, the variant of the OB-fold domain is fused to at least one of the heavy or light chain of an immunoglobulin monomer, preferably at the N- or C-terminus of the light or heavy chain. In another embodiment, the variant may be fused to both heavy or light chains.

In order to obtain such compounds, one can use a genetic construct comprising a DNA sequence selected from the group consisting of
a. the sequence coding for the heavy chain of an antibody fused, at its 3'end with the sequence coding for the variant of an OB-fold protein (potentially with an sequence coding for a linker)
b. the sequence coding for the heavy chain of an antibody fused, at its 5' end with the sequence coding for the variant of an OB-fold protein (potentially with an sequence coding for a linker)
c. the sequence coding for the light chain of an antibody fused, at its 3' end with the sequence coding for the variant of an OB-fold protein (potentially with an sequence coding for a linker)
d. the sequence coding for the light chain of an antibody fused, at its 5' end with the sequence coding for the variant of an OB-fold protein (potentially with an sequence coding for a linker).

This fusion can be made at the N-terminus and/or the C-terminus of the antibody chain (heavy and/or light chain). It is to be noted that, especially when using a small OB-fold domain (about 70 amino acids) such as a protein from the Sac7d family, it is possible to obtain a molecule that will have the structure of the antibody (two light chains paired to two heavy chains, and such dimers paired together), with the antibody regions, and further binding regions consisting of the modified OB-fold domain.

In a specific embodiment, the antibody part of the protein herein disclosed is a IgG molecule.

In another embodiment, the antibody part of the protein herein disclosed is a IgA molecule.

In another embodiment, the antibody part of the protein herein disclosed is a IgM molecule.

In another embodiment, the antibody part of the protein herein disclosed is a IgD molecule.

In another embodiment, the antibody part of the protein herein disclosed is a IgE molecule.

The antibody may be a human antibody, a rodent antibody (such as a mouse antibody or a rat antibody), a cat antibody, a dog antibody, a chicken antibody, a goat antibody, a camelid antibody (such as a camel antibody, a llama antibody, an alpaca antibody or a nanobody), a shark antibody, or an antibody from any other species. It may be a chimeric or humanized antibody. As reminded in Wikipedia, humanized antibodies are antibodies from non-human species whose protein sequences have been modified to increase their similarity to antibody variants produced naturally in humans. A chimeric antibody contains sequences from different species.

It is preferred when the antibody that is part of the molecule herein disclosed is an antibody that comprises two identical heavy chains (of about 400 to 500 amino acids, generally around 450 amino acids) and two identical light chains. Consequently, the antibody comprises the same Fab variable regions. This antibody is therefore a monospecific antibody where both parts (combination of light and heavy chains) of the antibody bind to the same epitope of an antigen.

However, the antibody may present different heavy and/or light chains. In particular, in some embodiments, the antibody is a bi-specific antibody. The term "antibody" thus encompasses both "classical antibodies" as disclosed above having identical heavy and light chains, but also engineered antibodies that have more than one specificity.

In a specific embodiment, the antibody presents one heavy and light chain from one antibody and another heavy and light chain from another antibody.

The antibody may bind to a target selected in the group consisting of:
Cell surface receptors: Insulin receptor, Low density lipoprotein receptor-related protein 1, Transferrin receptor, Epidermal growth factor receptor, Epidermal growth factor receptor variant III, Vascular endothelial growth factor receptor 1, Vascular endothelial growth factor receptor 2, Her2, Her3, Her4, PMSA, IGF-1R, GITR, RAGE, CD28.
Cell surface proteins: Mesothelin, EpCam, CD19, CD20, CD38, CD3, TIM-3, CEA, cMet, ICAM1, ICAM3, MadCam, a4b7, CD7, CD4, CD138.
Angiogenesis factors and Growth factors: VEGF, Angiopoietin 2, HGF, PDGF, EGF, GM-CSF, HB-EGF, TGF
Immune checkpoint inhibitors or activators: PD-1, PD-L1, CTLA4, CD28, B7-1, B7-2, ICOS, ICOSL, B7-H3, B7-H4, LAG3, KIR, 4-1BB, OX40, CD27, CD40L, TIM3, A2aR
Circulating proteins: TNFa, IL23, IL12, IL33, IL4, IL13, IL5, IL6, IL4, IFNg, IL17, RANKL, Bace1, alpha Synuclein, Tau, amyloid.
It is particularly envisaged when the antibody targets IL17 or a cell surface receptor in particular involved in cancer.

Hence are particularly foreseen
- A polypeptide comprising a variant as disclosed above, binding to PD-L1 and an antibody binding to HSP110
- A polypeptide comprising a variant as disclosed above, binding to HSP110 and an antibody binding to PD-L1 (such as atezolizumab, avelumab or durvalumab)
- A polypeptide comprising a variant as disclosed above, binding to PD-L1 and an antibody binding to EGFR (such as cetuximab, panitumumab, zalutumumab, nimotuzumab, and matuzumab).
- A polypeptide comprising a variant as disclosed above, binding to HSP110 and an antibody binding to EGFR

Alternatively, the polypeptide may contain a variant as disclosed above, and a biologically active molecule, such as an erythropoietin, an interferon, or etanercept.

In another embodiment, the variant of the OB-fold domain (in particular the variant of a protein of the Sac7d family) is conjugated to an organic molecule. This may be done by any method known in the art. In particular, one can chemically link the molecule to the protein. As a molecule, one can cite antiproliferative (cytotoxic and cytostatic agents) include cytotoxic compounds (e.g., broad spectrum), angiogenesis inhibitors, cell cycle progression inhibitors, PBK/m-TOR/AKT pathway inhibitors, MAPK signaling pathway inhibitors, kinase inhibitors, protein chaperones inhibitors, HDAC inhibitors, PARP inhibitors, Wnt/Hedgehog signaling pathway inhibitors, RNA polymerase inhibitors and proteasome inhibitors. One can also use anti-inflammatory molecules.

One can cite in particular DNA-binding or alkylating drugs such as anthracyclines (doxorubicin, epirubicin, idarubicin, daunorubicin) and its analogs, alkylating agents, such as calicheamicins, dactinomycines, mitromycines, pyrrolobenzodiazepines, and the like. One can also cite cell cycle progression inhibitors such as CDK inhibitors, Rho-kinase inhibitors, checkpoint kinase inhibitors, aurora kinase inhibitors, PLK inhibitors, and KSP inhibitors. One can also cite thalidomide and its derivatives lenalidomide and pomalidomide. In order for treating inflammation disorders, one can also use cyclooxygenase-2 inhibitors, 5-lipoxygenase inhibitors, quercetin and/or resveratrol as molecules conjugated to the polypeptide comprising the variant.

Interesting and preferred molecules are also disclosed above.

### Use of the variants

The variants can be used in particular in therapeutic methods, especially for treating cancer.

The invention thus relates to methods of treatment of cancer, comprising the administration of a therapeutic amount of a variant of an OB-fold herein disclosed (in particular a variant of a protein of the Sac7d family) to a subject in need thereof.

The term "therapeutic amount" or "effective amount", as used herein, is the amount sufficient to effect beneficial or desired results, such as clinical results, and an "effective amount" depends upon the context in which it is being applied. An effective amount is an amount that provides therapeutic improvement while minimizing side or adverse effect. Therapeutic improvement may be regression of tumor size in solid tumor, improvement in life quality of the subject, improvement of the efficacy of a combined treatment.

One can administer the variant by any method of the art.

In particular, one can inject the variant. In another embodiment, one can apply the variant topically (either on the skin or on the eye of the patient) as disclosed in WO 2014/173899. In another embodiment, one can administer the variant orally, as disclosed in WO 2016/062874.

The variants or polypeptides containing the variants can also be used in diagnostic methods. In particular, such variants or polypeptide may be linked to any marker known in the art and used in imagery methods. Indeed, PD-L1 is a marker of cancer cells, as is HSP110 which can be found at the cell surfaces. This is particularly interesting for detecting metastasis or following the efficacy of cancer treatment especially *in vivo.* One can administer such polypeptides of the invention that have bound to any imagery-detectable marker and follow the presence and binding of the administered molecules by imagery methods.

The invention thus also relates to a method for detecting the presence of, or for quantifying, a subunit of a multimeric protein in a sample, comprising the step of
a. Exposing the sample to a variant as disclosed, that binds to the subunit but not the fully formed multimeric protein, in such conditions that such binding is possible
b. Recovering the variant and/or detecting, or measuring the amount of, quantifying, ELISA, fluorescence, columns] the subunit.

The recovery of b) can be perfomed by various washes or methods common in the art. The detection or quantification can be performed by any method such as ELISA or other methods in the art.

### DESCRIPTION OF THE FIGURES

Figure 1: alignment of proteins of the Sac7d family.
Figure 2: binding of B11 to both human (upper curve) and mouse (lower curve) proteins.
Figure 3: inhibition of binding of PD1 and PD-L1 using a variant (B11) protein binding to PD-L1.
Figure 4: inhibition of binding of PD1 and PD-L1 using a variant (B11) protein binding to PD-L1, alone (triangles) or as a dimer (reverse triangles).
Pembrolizumab (squares) and atezolizumab (circles) were used as controls.
Figure 5: mean tumor weight (mg) measured in the different experimental groups at the end of study. NF1 = anti-HSP110 variant. NF2: anti-PD-L1 variant. NF1-NF2: dimer of both variants.
Figure 6: Data analysis of chicken CD3 expression in tumor.
Figure 7: data analysis of chicken MMD expression in tumor.
Figure 8: number of dead and surviving embryos at the end of the study.
Figure 9: mean tumor weight (mg) measured in the different experimental groups after 10 days of treatment on the CAM. Mean values ± SEM of tumor weight measured for each experimental group.
Figure 10: mean tumor weight (mg) measured in the different experimental groups after 10 days of treatment on the CAM. Mean values ± SEM of tumor weight measured for each experimental group.
Figure 11: mean tumor weight (mg) measured in the different experimental groups at the end of study. Mean values ± SEM of tumor weight (mg).
Figure 12: Relative Quantity of chicken CD3 Expression (± SEM) in Tumor per Group.
Figure 13: data analysis of chicken MMD expression in tumor. Relative Quantity of chicken MMD Expression (± SEM) in Tumor per Group.

### EXAMPLES

### Example 1. Characterization of a variant of Sac7d binding to PD-L1

Efficacy of the PDL1 neutralizing Nanofitins was evaluated using the PD1/PDL1 blockade bioassay from Promega. The assay consists of two genetically engineered cell lines provided in a thaw and use format: Jurkat T cells expressing human PD-1 and a luciferase reporter driven by an NFAT response element (PD-1 Effector Cells) and CHO-K1 cells expressing human PD-L1 and an engineered cell surface protein designed to activate cognate TCRs in an antigen-independent manner (PD-L1 aAPC/CHO-K1 Cells). When the two cell types are co-cultured, the PD-1/PD-L1 interaction inhibits TCR signaling and NFAT-RE-mediated luminescence. Neutralization of the PD1/PDL1 interaction releases the inhibitory signal and results in TCR activation and NFAT-RE-mediated luminescence.
Briefly, 0.5 mL of thaw-and-use PD-L1 aAPC/CHO-K1 cells suspension was transfered to 14.5 mL of cell recovery medium (90% Ham's F12/10% FBS) and mixed well by gently inverting 1-2 times. 100 µL of the cell suspension were dispensed to each of the inner 60 wells of two 96-well, white, flat-bottom assay plates. 100 µL of cell recovery medium were added to each of the outside wells of the assay plates. The assay plates was then covered with a lid, and incubated for 16-20 hours in a 37°C, 5% CO2 incubator. After overnight incubation, the medium was removed by flicking the plate. 40 µL of the test item concentration ranges and 40 µL of the thaw and use PD-1 effector cells suspension, all prepared in assay buffer (99% RPMI1640/1% FBS), were immediatly added to the inner wells. Subsequently, 80 µL of assay buffer were added to the outside wells and the plate was covered and incubated for six hours in a 37°C, 5% CO2 incubator. After the incubation period, the plate was allowed to equilibrate to ambient temperature for 5-10 minutes. 80 µL of Bio-Glo™ Reagent were added to the inner 60 wells of the assay plates and the plate was allowed to incubate at ambient temperature for 5-30 minutes. Luminescence was finally measured and data analysis was performed on GraphPad Prism software V6.

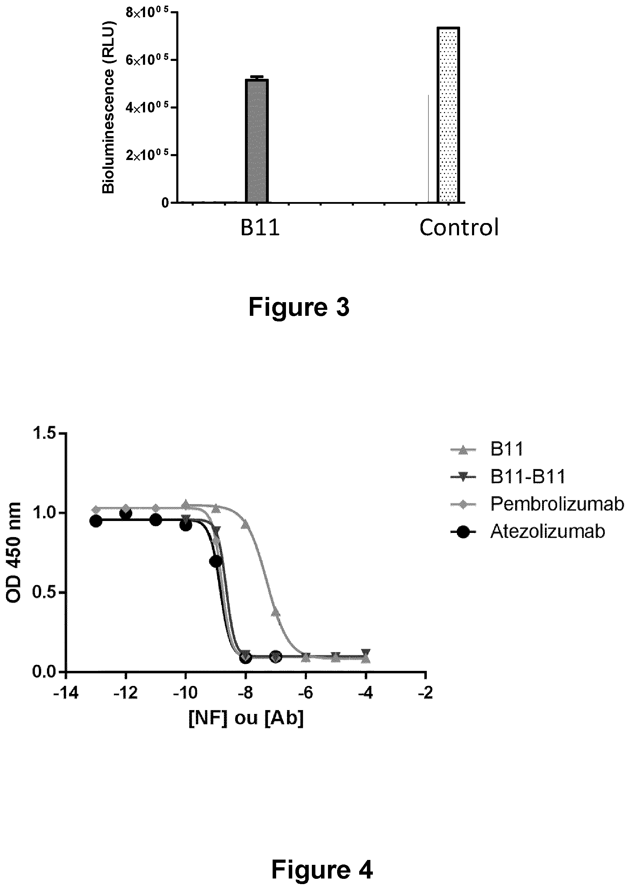

A Sac7d variant (B11, SEQ ID NO: 21) was found to inhibit PD1/PD-L1 binding (Figure 3). A correlation between affinity and efficacy of neutralization was also observed (not shown). A dimer of B11 was also shown to be as efficient as controls (Figure 4). B11 is also named NF2 in the examples.
This variant was also found to interact with both the human and mouse PD-L1 proteins (figure 2). This property is of particular interest as such species crossing is not observed often for PD-L1 binders, and is useful for pre-clinical studies.
Affinity of B11 to PD-L1 proteins (Surface Plasmon Resonance) is about K_{D} = 1.5x10⁻⁸ M (human) and K_{D} = 3.2x10⁻⁸ M (mouse).

Maturation of the protein made it possible to determine that the key amino acids that need to be present for obtaining binding to human PD-L1 are 18, I26, L31, M42 and L44. R24, Y29, K33 are further needed for binding to mouse PD-L1.

### Example 2. Characterization of a variant of Sac7d binding to HSP110

A Sac7d variant (A-C2, SEQ ID NO: 27) was found to bind to HSP110 and to inhibit HSP110-Stat3 binding.
A-C2 favored expansion of anti-tumoral M1 macrophages. A-C2 is also named NF1 in the examples.

Maturation of the protein made it possible to determine that the key amino acids that need to be present for obtaining binding to human PD-L1 are W8, R9, W21, D22, W24, Y31, H33, M40 and K44.

### Example 3. In vivo experiments - anti-HSP110 variant alone or in combination with anti-PD-L1 variant

### Material and methods

All *in vivo* experiments were performed using the CAM technology, corresponding to the graft of cancer cells onto the chorioallantoic membrane (CAM) of chick embryos (see Kroiss et al, Oncogene. 2015 May 28;34(22):2846-55; Green et al, PLoS One. 2009 Aug 21;4(8):e6713).
MDA-MB-231 human breast carcinoma cell line were grafted on the CAM of 9-days old chick embryos, with treatments between days 10 and 17 (1 to 8 days prior to grafting, followed by analysis for toxicity and tumor growth inhibition.
In brief, fertilized White Leghorn eggs were incubated at 37.5°C with 50% relative humidity for 9 days. At that moment (E9), the chorioallantoic membrane (CAM) was dropped down by drilling a small hole through the eggshell into the air sac, and a 1 cm² window was cut in the eggshell above the CAM. At least 20 eggs (depending on embryo surviving rate after 9 days of development, there could be more than 20 eggs per group) were used for each group. An inoculum of 1.10⁶ MDA-MB-231 cells was added onto the CAM of each egg

Keytruda® (pembrolizumab) was used as the reference compound. Six experimental groups as described in Table 2.

**Table 2. Study groups**

| Group | Description | Compound | Dose (mg/kg) |
|---|---|---|---|
| 1 | Neg. Ctrl. (Vehicle) | PBS | --- |
| 2 | Pos. Ctrl. | Keytruda® | 2 |
| 3 | Exp. Cpd. 1 [1] | A-C2 (NF1) | 2 |
| 4 | Exp. Cpd. 1 [2] | A-C2 | 20 |
| 5 | Exp. Cpd. 2 [1] | A-C2-B11 | 2 |
| 6 | Exp. Cpd. 2 [2] | A-C2-B11 | 20 |

On day E10, tumors began to be detectable. Treatments corresponding to different groups are detailed in Table 3. After each treatment, eggs were individually checked daily for death or visible abnormalities (visual checks).

**Table 3. Description of treatment groups**

| Group | Description | Treatment | Injected [C] (in 100 µl/egg) | Final [C] (in ovo) | Dosing Regimen |
|---|---|---|---|---|---|
| 1 | Neg. Ctrl. | PBS | --- | --- | E10, E12, E14, E15, E17 |
| 2 | Keytruda | Keytruda® | 0.12 mg/100 µl | 2 mg/kg | E10, E12, |
| | | | | | E14, E15, E17 |
| 3 | NF1 - 2 mg/kg | NF1 | 0.12 mg/100 µl | 2 mg/kg | E10, E12, E14, E15, E17 |
| 4 | NF1 - 20 mg/kg | NF1 | 1.2 mg/100 µl | 20 mg/kg | E10, E12, E14, E15, E17 |
| 5 | NF1-NF2 - 2 mg/kg | NF1-NF2 | 0.12 mg/100 µl | 2 mg/kg | E10, E12, E14, E15, E17 |
| 6 | NF1-NF2 - 20 mg/kg | NF1-NF2 | 1.2 mg/100 µl | 20 mg/kg | E10, E12, E14, E15, E17 |

### Quantitative Evaluation of Tumor Growth

On day E18, the upper portion of the CAM (with tumor) was removed from all viable embryos with tumors, washed by PBS buffer and then directly transferred in PFA (fixation for 48 hrs.). After that, tumors were carefully cut away from normal CAM tissue and weighed. A one-way ANOVA analysis with post-tests is performed on the data.

### Quantitative Evaluation of immune Cell Infiltration

On day E18, 6 tumor samples per group were collected to evaluate the infiltration of immune cells. Each tumor sample was removed, washed by PBS buffer and then directly transferred in 4% PFA (fixation for 48 hr). After that, genomic RNA was extracted from fixed tumor (commercial kit) and analyzed by RT-qPCR with specific primers for chicken CD3 and MMD sequences.
For all points done in qPCR, expression of GAPDH is also analyzed, as reference gene expression, and used to normalize immune biomarker expression between samples. A one-way ANOVA analysis with post-tests is done on the data.

### Quantitative Evaluation of Embryonic Toxicity

Embryonic viability was checked daily. The number of dead embryos was also counted on E18, in combination with the observation of eventual visible gross abnormalities, to evaluate treatment-induced embryo toxicity.

### Significance of Statistical Analysis

For all analyses, statistical difference between groups are made visible on graphs by the presence of stars with the following meaning:
- No star: No statistical difference (p value > 0.05);
- *: 0.05 ≥ p-value > 0.01;
- **: 0.01 ≥ p-value > 0.001;
- ***: 0.001 ≥ p-value.

### Results

### Quantitative Evaluation of Tumor Growth

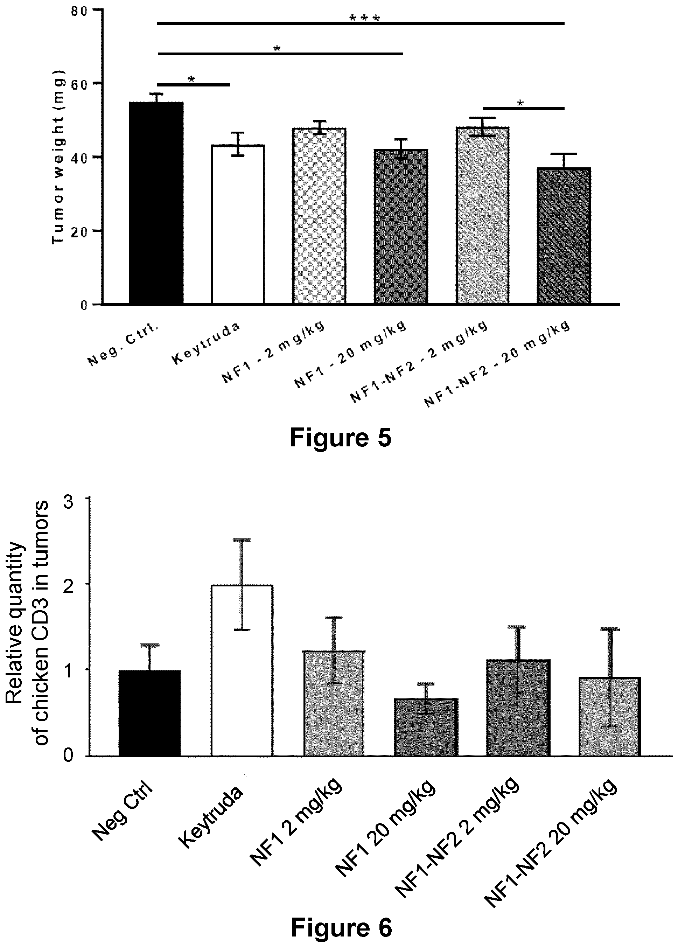

Figure 5 presents the mean tumor weight (mg) measured in the different experimental groups at the end of study.

### Quantitative Evaluation of immune Cell Infiltration

### Chicken CD3 expression in tumor

Figure 6 present data analysis of data analysis of chicken CD3 expression in tumor.

### Chicken MMD expression in tumor

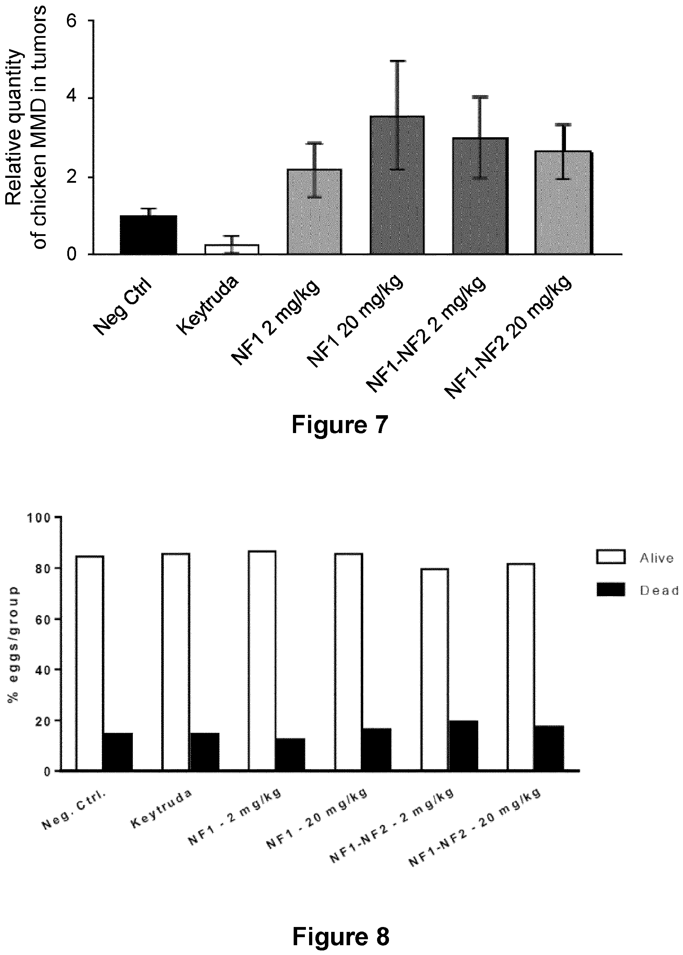

Figure 7 present data analysis of chicken MMD expression in tumor.

### Quantitative Evaluation of Embryonic Toxicity

Figure 8 present the number of dead and surviving embryos at the end of the study.

### Conclusion

The aim of this project was to evaluate the toxicity and efficacy of an anti-HSP110 (NF1, A-C2) alone or as a dimer with an anti-PD-L1 (NF2, B11) against tumors initiated from MDA-MB-231 human breast carcinoma cell line in a CAM Model. Keytruda® was used as the Reference compound.

In terms of tumor growth, when compared to the Negative Control, both NF1 and NF1-NF2 compounds induced a significant tumor growth inhibition at the high dose (20 mg/kg). The effect of NF1-NF2 compound is dose dependent, its performance at 20 mg/kg is evidently better than at 2 mg/kg.
In terms of immune cells filtration in tumor, an increase tendency of CD3 positive cells infiltration was observed in presence of Keytruda; an increase tendency of MMD positive cells was observed in presence of the tested compounds, with a dose effect for NF1 alone. All these increases are not statistically significant. Finally, treatments with both NF1 and NF1-NF2 compounds did not induce any evident embryonic toxicity at all tested doses when compared to Negative Control.

### Example 4. Study of efficacy of NF-B11 compound on breast carcinoma tumors initiated from MDA-MB-231 cell lines

The CAM model as described in example 3 was used.

### Treatments

At day 10 (E10), tumors began to be detectable. They were then treated during 10 days, every two days (E11, E13, E15, E17) by dropping 100 µl of vehicle (in PBS), Ref Compound (Tecentriq/ Atezolizumab), and NF-B11 at two different doses onto the tumor (see Table 4 for concentration).

**Table 4. Experiments**

| **Group description** | **Molecule Name** | | **Concentration** |
|---|---|---|---|
| **Group 1** | Negative Ctrl (vehicle) | Neg Ctrl | PBS |
| **Group 2** | Positive Ctrl (Ref. Compound) | Tecentriq | 1,02µg/mL |
| **Group 3** | Exp. Group 2 | NF-B11 10XIC50 | 41µg/mL |
| **Group 4** | Exp. Group 3 | NF-B11 100XIC50 | 410µg/mL |

### Results

### Tumors Growth

Table 5 presents the mean values of the tumor weights for the different experimental groups at E18.

**Table 5. Mean value, SD, SEM and p-value of tumor weight (mg) for each experimental group**

| | **n** | **Tumor weight (mg)** | **SD** | **SEM** | **% of tumor regression** |
|---|---|---|---|---|---|
| Neg Ctrl | 12 | 12,19 | 6,88 | 1,98 | N/A |
| Tecentriq | 12 | 9,92 | 3,34 | 0,96 | 18,62 |
| NF-B11 10XIC50 | 12 | 9,25 | 3,72 | 1,07 | 24,10 |
| NF-B11 100XIC50 | 11 | 6,45 | 2,44 | 0,73 | 47,06 |

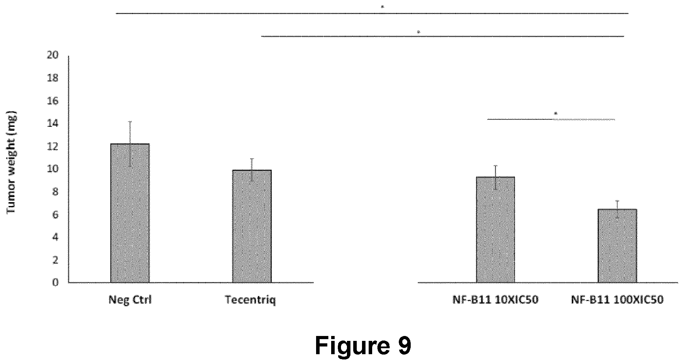

Figure 9 presents the mean tumor weight (mg) measured in the different experimental groups after 10 days of treatment on the CAM.

### Toxicity

Table 6 presents the number of dead and surviving embryos after 10 days of treatment in the different experimental groups.

**Table 6. Number of dead and surviving embryo for each experimental group.**

| | **Total** | **Alive** | **Dead** | **% alive** | **% dead** |
|---|---|---|---|---|---|
| Neg Ctrl | 16 | 11 | 5 | 69 | 31 |
| Tecentriq | 16 | 12 | 4 | 75 | 25 |
| NF-B11 10XIC50 | 16 | 12 | 4 | 75 | 25 |
| NF-B11 100XIC50 | 16 | 12 | 4 | 75 | 25 |

In term of results, Tecentriq has no statistical effect compared to negative control while NF-B11 treatment shows a 47% reduction of tumor weight, for the treatment at 50 times the IC50 respectively. For the dose of 10 times the IC50, the tumor reduction is around 24% but is not statistically different compare to negative control.

### Example 5. Study of efficacy of NF-B11 compound on breast carcinoma tumors initiated from MDA-MB-231 cell lines

### Treatments

At day 10 (E10), tumors began to be detectable. They were then treated during 10 days, every two days (E11, E13, E15, E17) by dropping 100 µl of vehicle (in PBS), Ref Compound (Tecentriq), NF-B11 or NF-B11-B11 compounds (see Table 7 for concentration).

**Table 7. Groups for the study**

| | **Group description** | **Molecule Name** | **Final Concentration** |
|---|---|---|---|
| **Group 1** | Negative Ctrl (vehicle) | Neg Ctrl | 0 |
| **Group 2** | Positive Ctrl (Ref. Compound) | Tecentriq | 200x IC50 |
| **Group 3** | NF-B11 100xIC50 | NF-B11 | 100xlC50 |
| **Group 4** | NF-B11-B11 10x IC50 | NF-B11-B11 10x | 10x IC50 |
| **Group 5** | NF-B11-B11 100x IC50 | NF-B11-B11 100x | 100x IC50 |

### Tumor growth analysis

At day 18 (E18) the upper portion of the CAM was removed, washed in PBS and then directly transferred in PFA (fixation for 48h). The tumors were then carefully cut away from normal CAM tissue. Tumors were then weighed. A one-way ANOVA analysis with post-tests has been done on these data.

### Significance on statistical analysis

For tumor weight and metastasis, statistical difference between groups are visible on graphs by presence of stars with the following signification:
- No stars: no statistical different (p value > 0.05);
- One star (*): 0.05 > p value > 0.01;
- Two stars (**): 0.01 ≥ p value > 0.001;
- Three stars (***): 0.001 ≥ p value.

### Toxicity

The number of dead embryo evaluates the acute toxicity after 10 days of the treatment.

### Results

### Tumors Growth

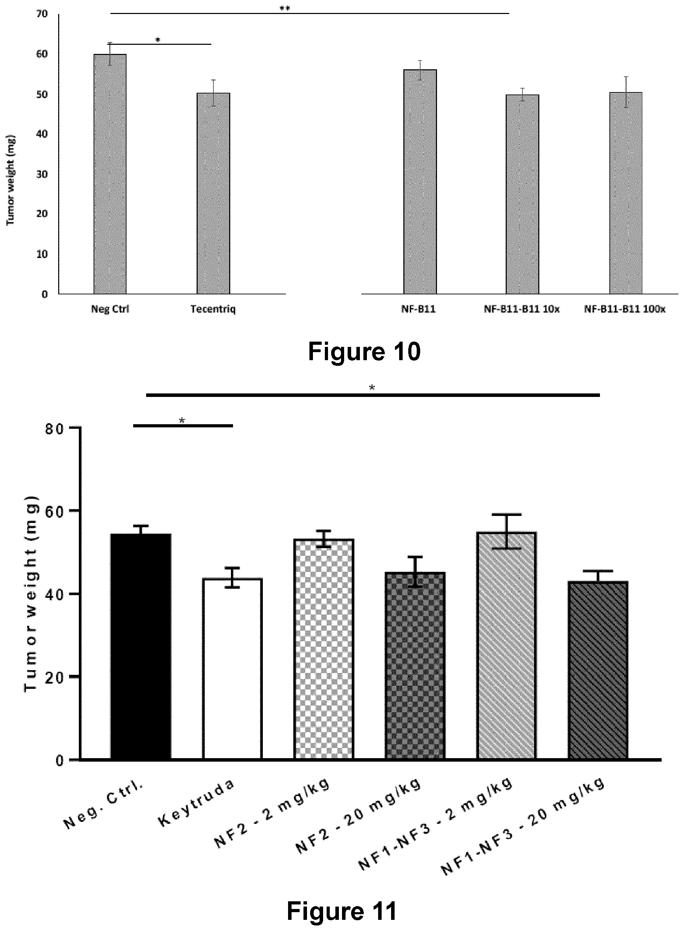

Figure 10 presents the mean tumor weight (mg) measured in the different experimental groups after 10 days of treatment on the CAM.

### Toxicity

Table 8 presents the number of dead and surviving embryos after 10 days of treatment in the different experimental groups.

**Table 8 Number of dead and surviving embryo for each experimental group.**

| | **Total** | **Alive** | **Dead** | **% alive** | **% dead** |
|---|---|---|---|---|---|
| Neg Ctrl | 18 | 18 | 0 | 100 | 0 |
| Tecentriq | 16 | 13 | 3 | 81 | 19 |
| NF-B11 | 18 | 17 | 1 | 94 | 6 |
| NF-B11-B11 10x | 17 | 13 | 4 | 76 | 24 |
| NF-B11-B11 100x | 18 | 17 | 1 | 94 | 6 |

The study tested the efficacy of anti-PD-L1 (NF-B11) compounds on tumor initiated from MDA-MB-231 cells.
In this study, Tecentriq, and NF-B11-B11 at 10 times IC50 have a significant effect on tumor weight (16-17 % reduction).
Concerning toxicity, the ratio of dead embryo is higher in treated group NF-B11-B11 at 10 time IC50, but is lower in treated group NF-B11-B11 at 100X IC50 showing that there is no specific toxicity due to treatment (at the doses tested).

### Example 6. Evaluation of In Vivo Toxicity and Efficacy of 2 Compounds in Tumors Derived from MDA-MB-231 Human Breast Cell Line in a CAM Model

**Table 9. Compounds and concentration**

| **Group** | **Description** | **Compound** | **Dose (mg/kg)** |
|---|---|---|---|
| **1** | Neg. Ctrl. (Vehicle) | PBS | --- |
| **2** | Pos. Ctrl. | Keytruda® | 2 |
| **3** | Exp. Cpd. 1 [1] | NF2 | 2 |
| **4** | Exp. Cpd. 1 [2] | NF2 | 20 |
| **5** | Exp. Cpd. 2 [1] | NF1-NF3 | 2 |
| **6** | Exp. Cpd. 2 [2] | NF1-NF3 | 20 |

NF1: anti-HSP110 Sac7d variant (A-C2)
NF2 : anti-PD-L1 sac7d variant (B11)
NF3 : variant of Sac7d binding to EGFR

### Treatments

On day E10, tumors began to be detectable. Treatments corresponding to different groups are detailed in Table 10. After each treatment, eggs were individually checked daily for death or visible abnormalities (visual checks).

**Table 10. Description of treatment groups.**

| **Group** | **Description** | **Treatment** | **Injected [C] (in 100 µl/egg)** | **Final [C] (in ovo)** | **Dosing Regimen** |
|---|---|---|---|---|---|
| **1** | **Neg. Ctrl.** | PBS | --- | --- | E10, E12, E14, E15, E17 |
| **2** | **Keytruda** | Keytruda® | 0.12 mg/100 µl | 2 mg/kg | E10, E12, E14, E15, E17 |
| **3** | **NF2-2 mg/kg** | NF2 | 0.12 mg/100 µl | 2 mg/kg | E10, E12, E14, E15, E17 |
| **4** | **NF2 - 20 mg/kg** | NF2 | 1.2 mg/100 µl | 20 mg/kg | E10, E12, E14, E15, E17 |
| **5** | **NF1-NF3 - 2 mg/kg** | NF1-NF3 | 0.12 mg/100 µl | 2 mg/kg | E10, E12, E14, E15, E17 |
| **6** | **NF1-NF3 - 20 mg/kg** | NF1-NF3 | 1.2 mg/100 µl | 20 mg/kg | E10, E12, E14, E15, E17 |

### Results

### Quantitative Evaluation of Tumor Growth

Figure 11 presents the mean tumor weight (mg) measured in the different experimental groups at the end of study. Mean values ± SEM of tumor weight (mg).

### Quantitative Evaluation of immune Cell Infiltration

### Chicken CD3 expression in tumor

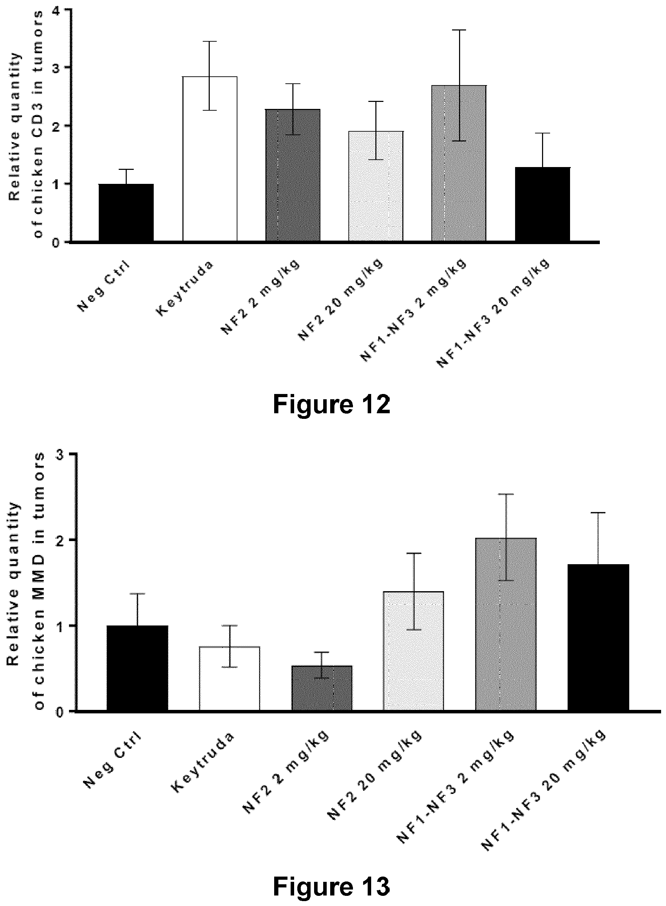

Figure 12 present data analysis of chicken CD3 expression in tumor.

### Chicken MMD expression in tumor

Figure 13 present data analysis of data analysis of chicken MMD expression in tumor.

### Quantitative Evaluation of Embryonic Toxicity

Table 11 present the number of dead and surviving embryos at the end of the study.

**Table 11. Number of dead and alive embryos per group**

| **Group** | **Description** | **Total** | **Alive** | **Dead** | **%Alive** | **% Dead** |
|---|---|---|---|---|---|---|
| **1** | **Neg. Ctrl.** | 23 | 17 | 6 | 73,91 | 26,09 |
| **2** | **Keytruda** | 20 | 14 | 6 | 70,00 | 30,00 |
| **3** | **NF2 - 2 mg/kg** | 24 | 15 | 9 | 62,50 | 37,50 |
| **4** | **NF2 - 20** | 21 | 14 | 7 | 66,67 | 33,33 |
| | **mg/kg** | | | | | |
| **5** | **NF1-NF3 - 2 mg/kg** | 24 | 16 | 8 | 66,67 | 33,33 |
| **6** | **NF1-NF3 - 20 mg/kg** | 21 | 14 | 7 | 66,67 | 33,33 |

First, in terms of tumor growth, when compared to the Negative Control, both NF2 and NF1-NF3 compounds didn't impact tumor growth at the low dose (2 mg/kg); at the high dose (20 mg/kg), we observed the tumor growth regression tendency for both compounds, but the tumor growth inhibition was significant only for NF1-NF3. Second, in terms of tumor immune cells filtration, we observed an increase tendency of CD3 positive cells infiltration in presence of Keytruda; an increase tendency of MMD positive cells in presence of the Sac7d variants, with a dose effect for NF2 alone. All these increases are not statistically significant.
Finally, treatments with both NF2 and NF1-NF3 compounds did not induce any evident embryonic toxicity at all tested doses when compared to Negative Control.

## Claims

1. A polypeptide comprising a variant of a protein of the Sac7d family, comprising SEQ ID NO: 16.

2. The polypeptide of claim 1, which comprises SEQ ID NO: 17 or SEQ ID NO: 18.

3. The polypeptide of claim 1, which comprises SEQ ID NO: 19, SEQ ID NO: 20, SEQ ID NO: 22, or SEQ ID NO: 23.

4. The polypeptide of any one of claims 1 to 3, which comprises SEQ ID NO: 21.

5. The polypeptide of any one of claims 1 to 4, wherein the variant of a protein of the Sac7d family binding to the subunit of a multimeric protein is linked or fused to another protein or polypeptide, in particular another variant of a protein of the Sac7d family.

6. The polypeptide of claim 5, wherein the other protein is a variant of the Sac7d family binds to HSP110 or to EGFR.

7. The polypeptide of claim 6, wherein said other variant comprises SEQ ID NO: 24, SEQ ID NO: 25, SEQ ID NO: 26, SEQ ID NO: 27, SEQ ID NO: 28 or SEQ ID NO: 29.

8. The polypeptide of any one of claims 1 to 7, which comprises the sequence SEQ ID NO: 21 and SEQ ID NO: 27.

9. The polypeptide of any one of claims 1 to 8, which is conjugated to an organic molecule.

10. A genetic construct comprising a DNA sequence coding for the polypeptide of any one of claims 1 to 8.

11. A method for producing the polypeptide of any one of claims 1 to 8, comprising the steps consisting of
a. Culturing a cell culture wherein the cells have been transformed by a genetic construct of claim 10,
and
b. Recovering the polypeptide.

12. A polypeptide according to any one of claims 1 to 9 as a medicament.

13. A polypeptide according to any one of claims 1 to 9, for use thereof for the treatment of cancer, in particular a non-small cell lung carcinoma, an urothelial carcinoma, a gastric cancer, a liver cancer, a kidney cancer, a head and neck squamous cell cancer, an esophageal squamous cell carcinoma, a primary mediastinal B-cell lymphoma, a classical Hodgkin lymphom, a melanoma, a merkel cell carcinoma, a cervical cancer, a microsatellite instability-high cancer, a bladder cancer, a breast cancer, a small cell lung cancer, a colorectal cancer, a pancreatic cancer or a prostate cancer.

14. The polypeptide for use according to claim 13, in combination with chemotherapy or treatment with CAR-T cells.

15. Composition containing a polypeptide according to any one of claims 1 to 13 and a chemotherapy agent or CAR-T cells for simultaneous, separate or sequential (spread out over time) use in the treatment of cancer, in particular a non-small cell lung carcinoma, an urothelial carcinoma, a gastric cancer, a liver cancer, a kidney cancer, a head and neck squamous cell cancer, an esophageal squamous cell carcinoma, a primary mediastinal B-cell lymphoma, a classical Hodgkin lymphom, a melanoma, a merkel cell carcinoma, a cervical cancer, a microsatellite instability-high cancer, a bladder cancer, a breast cancer, a small cell lung cancer, a colorectal cancer, a pancreatic cancer or a prostate cancer.
